# EUROPEAN PATENT APPLICATION

(11) **EP 4 487 837 A1**
(43) Date of publication of application: **08.01.2025**
(21) Application number: 23186744.1
(22) Date of filing: 20.07.2023
(51) Int. Cl.: A61K 9/00, A61K 31/137, A61K 31/56, A61K 45/06, A61P 11/06

(54) **AN INHALABLE FORMULATION OF FLUTICASONE PROPIONATE AND ALBUTEROL SULFATE FOR THE TREATMENT OF ASTHMA**

(30) Priority: 07.07.2023 US 202363525562 P; 07.07.2023 US 202363525601 P
(71) Applicant: Norton (Waterford) Limited, Waterford, X91 WK68 (IE)
(72) Inventor: The designation of the inventor has not yet been filed
(74) Representative: Elkington and Fife LLP

(57) **Abstract**

This invention relates to a method for the treatment of asthma comprising pro re nata (PRN) administration of a fixed-dose dry powder inhalation composition comprising fluticasone propionate and albuterol sulfate as a rescue medication in patients aged from ≥4 years to <18 years. Also provided is a fixed-dose dry powder inhalation composition and dry powder inhaler for performing the method of the present invention.

## Description

### Background of the invention

This invention relates to an inhalable formulation, and particularly to a fixed-dose composition, of fluticasone propionate and albuterol sulfate for the treatment of asthma.

Asthma is one of the most common chronic diseases. It is a heterogeneous condition that affects the airways of the lungs and is characterized by airway inflammation and bronchial hyper-responsiveness, resulting in variable reductions in expiratory airflow that vary over time in their occurrence, frequency, and intensity. During acute asthmatic episodes, the airway passages become narrower and more obstructed, resulting in coughing, wheezing, tightness of the chest, shortness of breath, and increased mucus production. In some subjects, asthma may lead to chronic irreversible changes in airway structure and function, increasing morbidity and mortality.

Inhaled corticosteroids and β₂-agonists represent two classes of active ingredient that have been developed to treat respiratory disorders, particularly asthma. Each class has differing targets and effects.

Inhaled corticosteroids (ICSs) are steroid hormones used in the long-term control of respiratory disorders. They function by reducing the airway inflammation, controlling symptoms and reducing future risks of exacerbation and lung function decline. They are often termed "controller" or "maintenance" medicines.

Presently marketed ICSs are for example beclomethasone dipropionate, budesonide, ciclesonide, fluticasone furoate, fluticasone propionate and mometasone furoate. The compounds are all well-known in the art. For example, fluticasone propionate is named as S-(fluoromethyl)-6α,9-difluoro-11β,17-dihydroxy-16α-methyl-3-oxoandrosta-1,4-diene-17β-carbothioate-17-propanoate.

Short-acting β₂-agonists (SABAs) are examples of bronchodilators, and are employed to dilate the bronchi and bronchioles, decreasing resistance in the airways, and thereby increasing the airflow to the lungs. Bronchodilators may be short-acting or long-acting. Short-acting bronchodilators provide a rapid but short-term relief from acute bronchoconstriction (and are often called "rescue" or "reliever" medicines), whereas long-acting bronchodilators typically help control symptoms over a longer period of time compared to SABAs.

SABAs are used acutely to provide rapid symptom relief and can be lifesaving, but they do not address underlying airway inflammation. When patients experience worsening asthma symptoms, they gain immediate relief with SABA medication and therefore increase its use. However, patients generally do not increase the use of ICS in a similar manner. SABA overuse, defined as use of ≥3 canisters per year is common and is a risk factor for poor asthma outcomes and severe exacerbation.

Albuterol (also known as salbutamol) is a short-acting β₂-agonist that is indicated for the treatment or prevention of bronchospasm in patients with asthma. It is named as 4-[2-(tert-butylamino)-1-hydroxyethyl]-2-(hydroxymethyl)-phenol. Albuterol is the usual bronchodilator used in acute asthma management. It is typically administered as the sulfate salt, the structure of which is well-known in the art.

According to the Global Initiative for Asthma (GINA) 2023 guidelines, a step-wise approach is taken to treatment. For adults and adolescents (aged ≥12 years), the approach is divided into track 1 and track 2.

In track 1, at steps 1-2, which represent a mild form of asthma, the patient is given an as-needed low-dose ICS-formoterol (a long-acting β₂ adrenoceptor agonist (LABA) with a rapid onset of action). At step 3, a maintenance low-dose ICS-formoterol is given. At step 4, this is increased to a medium-dose ICS-formoterol. At step 5, an add on LAMA (long-acting muscarinic antagonist) is included, and a high-dose ICS-formoterol is considered. Over all steps, the reliever is an as-needed low-dose ICS-formoterol. In track 2, the reliever is an as-needed SABA or an as-needed ICS-SABA. Step 1 is to take an ICS whenever the SABA is taken. Step 2 adds a low-dose maintenance ICS. Step 3 is a low-dose maintenance ICS-LABA. Step 4 is a medium/high-dose ICS-LABA. At step 5, an add on LAMA is included, and a high-dose ICS-LABA is considered.

A similar approach for pediatric patients (aged 6-11) is recommended in the GINA guidelines. Step 1 is to take an ICS whenever the SABA is taken. Step 2 adds a low-dose maintenance ICS. Step 3 is a low-dose maintenance ICS-LABA, or medium-dose maintenance ICS, or very low dose ICS-formoterol maintenance and reliever (MART). Step 4 is a medium-dose ICS-LABA or low dose ICS-formoterol maintenance and reliever (MART). At step 5, a high-dose ICS-LABA or add-on therapy is considered. The reliever is an as-needed SABA or low-dose ICS-formoterol for MART in steps 3 and 4.

Interestingly, despite the availability of the present therapies the majority of asthmatic patients remain poorly controlled. There are several likely contributing causes of poor control one of which is poor adherence to prescribed maintenance therapies. This is a contributor to excess morbidity and mortality of asthma.

Despite the benefits of regular daily maintenance treatment, poor adherence is commonplace and associated with significant asthma-related morbidity. Most patients with persistent asthma take for example ICS therapy infrequently, only using 2 to 4 canisters per year.

Recently, a FDC of budesonide and albuterol sulfate was evaluated in a randomized clinical trial as a rescue medication for asthma at two dose strengths (A. Papi et al., "Albuterol-Budesonide Fixed-Dose Combination Rescue Inhaler for Asthma", The New England Journal of Medicine, 2022, 386(22), 2071-2083). The study was entitled "A Study to Assess the Efficacy and Safety of Budesonide/Albuterol Metered-dose Inhaler (BDA MDI/PT027) in Adults and Children 4 Years of Age or Older With Asthma (MANDALA)", and was performed under ClinicalTrials.gov Identifier: NCT03769090.

Adults and adolescents (≥12 years of age) were randomly assigned in a 1:1:1 ratio to one of three trial groups, namely: a fixed-dose combination of budesonide 160 mcg/albuterol 180 mcg (with each delivered dose consisting of two actuations of 80 mcg and 90 mcg, respectively) (the "higher-dose" combination group); a fixed-dose combination of budesonide 80 mcg/albuterol 180 mcg (with each delivered dose consisting of two actuations of 40 mcg and 90 mcg, respectively) (the "lower-dose" combination group); or 180 mcg of albuterol (with each delivered dose consisting of two actuations of 90 mcg) (the "albuterol-alone" group).

Children 4 to 11 years of age were randomly assigned to only the lower-dose combination group or the albuterol-alone group. The primary efficacy end point was the first event of severe clinical asthma exacerbation in a time-to-event analysis, which was performed in the intention-to-treat population.

Inconsistent results were demonstrated for the low dose group across age groups. Patients in the 4-11 years old age group failed to demonstrate a treatment response while the results were paradoxical for patient 12-18 demonstrating a trend to the treatment in the low dose group but favoring active control in the high dose group. Thus, the low dose was not approved even for adults, and no treatment was approved for patients aged 4-17-year-olds.

Thus, there remains a need in the art for improving the treatment of pediatric and adolescent asthma patients, i.e. patients 4 years to <18 years old which the present invention addresses.

### Summary of the invention

Accordingly, the present invention provides a method of treatment of asthma comprising a pro re nata (PRN) administration of a fixed-dose dry powder inhalation composition comprising fluticasone propionate and albuterol sulfate as a rescue medication in patients aged from ≥4 years to <18 years old.

The present invention also provides a fixed-dose dry powder inhalation composition comprising fluticasone propionate and albuterol sulfate for the treatment of asthma administered pro re nata (PRN) as a rescue medication in patients aged from ≥4 years to <18 years old.

In a preferred embodiment, the fixed-dose dry powder inhalation composition consists of fluticasone propionate, albuterol sulfate and lactose. Preferably, the lactose is alpha-lactose monohydrate.

The method of treatment of the present invention provides a significant improvement in the asthma treatment of pediatric and adolescent patients and is particularly surprising given the failure of the budesonide/albuterol fixed-dose combination in this patient population.

The invention will now be described in detail with reference to the accompanying drawings, in which:

### Brief description of the drawings

Fig. 1 is a front perspective view of a dry powder inhaler according to a preferred embodiment.;
Fig. 2 is a cross-sectional interior perspective view of the inhaler shown in Fig. 1;
Fig. 3 is an exploded perspective view of the example inhaler shown in Fig. 1;
Fig. 4 is an exploded perspective view of a top cap and electronics module of the inhaler shown in Fig.1;
Fig. 5 is an exploded isometric view of a deagglomerator according to the present disclosure;
Fig. 6 is a side elevation view of the deagglomerator of Fig. 5;
Fig. 7 is a top plan view of the deagglomerator of Fig. 5;
Fig. 8 is a bottom plan view of the deagglomerator of Fig. 5;
Fig. 9 is a sectional view of the deagglomerator of Fig. 5 taken along line 5'-5' of Fig. 6; and
Fig. 10 is a sectional view of the deagglomerator of Fig. 5 taken along line 6'-6' of Fig. 7.

### Detailed description

This invention relates to a method of treating asthma by administering a fixed-dose dry powder inhalation composition containing the APIs, fluticasone propionate and albuterol sulfate.

In its broadest embodiment, the indication being treated is asthma. In another preferred embodiment, the asthma treatment includes treating or preventing bronchospasm in patients 4 years to <18 years old with reversible obstructive airways disease. In yet another preferred embodiment, the asthma treatment includes preventing exacerbations in patients 4 years to <18 years old. Alternatively, the patients are aged 6 years to <18 years old.

In a preferred embodiment, the fixed-dose dry powder inhalation composition consists of fluticasone propionate, albuterol sulfate and lactose. Preferably, the lactose is alpha-lactose monohydrate.

The individual APIs are also known in the art and have already been discussed in the context of the background the present invention hereinabove.

The present invention relates to a fixed-dose combination (FDC), meaning that both APIs are present in the same formulation i.e., dry powder formulation. Alternatively, the two APIs can be present in a suspension or solution formulation. When referring to dry powder medicaments, both medicaments can be contained in a reservoir of the inhaler, in capsules or in blisters. Preferably, the two APIs are formulated together providing a fixed dose dry powder composition contained in the inhaler reservoir. In capsules or blister strips, the two APIs can be in the same or separate/different capsule/blister pocket as long as they are contained in the same device. Such inhalers are well known in the art, and are also described in more detail hereinbelow. It should be noted that when the medicament is contained in previously measured amounts in individual containers, e.g. a capsule or a blister, the metered dose may be termed a "pre-metered" dose.

The fixed dose combination of the present invention is administered pro re nata (PRN) as a rescue medicine, which means it is not used as maintenance therapy. Indeed, the patients can separately administer a maintenance dose of any kind of long-term asthma medication such as ICS, LABA, LAMA (long-acting muscarinic antagonist), SAMA (short-acting muscarinic antagonist), various biological medications and combinations thereof. The ICS can be the same as in the fixed-dose dry powder inhalation composition, i.e. fluticasone propionate, it can be another ICS, a combination of ICSs, or a combination of ICS with any of the above listed actives. Indeed, an advantage of the present invention is that the present combination may be used as rescue medication with any background asthma maintenance treatment or with no background treatment.

Examples of maintenance therapies which may be used with the present invention include ICSs, such as flunisolide, fluticasone propionate (approved products are the HFA pMDI and DPI e.g. ArmonAir^{®}/AirDuo^{®}), fluticasone furoate (approved product is the DPI), beclomethasone dipropionate (approved products are the DPI e.g. Easyhaler^{®} and the HFA pMDI, e.g. Qvar^{®}), budesonide (approved product is the DPI), mometasone furoate (approved products are the DPI and HFA pMDI), ciclesonide (approved product is the HFA pMDI) and combinations thereof.

Examples of LABAs include formoterol, arformoterol, salmeterol, bambuterol, clenbuterol and combinations thereof. Ultra-LABAs may also be used, e.g. abediterol, indacaterol, olodaterol, vilanterol, carmoterol and combinations thereof.

Examples of LAMAs include tiotropium, glycopyrronium, umeclidinium, aclidinium, darotropium and combinations thereof. As SAMA, an example is ipratropium.

Examples of biological maintenance therapies that may be used with the present invention include tezepelumab, dupilumab, mepolizumab, reslizumab, benralizumab, omalizumab, palivizumab and combinations thereof.

The maintenance therapy daily metered dose can be low-, medium- or high-dose depending on the type of medicament and patient's age. For example, for patients aged 12 years <18 years old, the ICS daily metered doses are as follows: Beclomethasone dipropionate, low-dose is 200-500 mcg, medium-dose is >500-1,000 mcg, high-dose is >1,000 mcg; Beclomethasone dipropionate ultra-fine particle, low-dose is 100-200 mcg, medium-dose is >200-400 mcg, high-dose is >400 mcg; Budesonide, low-dose is 200-400 mcg, medium-dose is >400-800 mcg, high-dose is >800 mcg; Ciclesonide, low-dose is 80-160 mcg, medium-dose is >160-320 mcg, high-dose is >320 mcg; Fluticasone furoate, low-dose and medium-dose are 100 mcg, high-dose is 200 mcg; Fluticasone propionate, low-dose is 100-250 mcg, medium-dose is >250-500 mcg, high-dose is >500 mcg and Mometasone furoate, low-dose and medium-dose are 200-400 mcg, high-dose is >400 mcg.

For patients aged 6 to <12 years old the ICS daily metered doses are as follows. Beclomethasone dipropionate, low-dose is 100-200 mcg, medium-dose is >200-400 mcg, high-dose is >400 mcg. Beclomethasone dipropionate ultra-fine particle, low-dose is 50-100 mcg, medium-dose is >100-200 mcg, high-dose is >200 mcg. Budesonide, low-dose is 100-200 mcg, medium-dose is >200-400 mcg, high-dose is >400 mcg. Budesonide nebules, low-dose is 250-500 mcg, medium-dose is >500-1,000 mcg, high-dose is >1,000 mcg. Ciclesonide, low-dose is 80 mcg, medium-dose is >80-160 mcg, high-dose is >160 mcg. Fluticasone furoate, low-dose and medium-dose are 50 mcg. Fluticasone propionate, low-dose is 50-100 mcg, medium-dose is >100-200 mcg, high-dose is >200 mcg. Mometasone furoate, low-dose and medium-dose are 100 mcg, high-dose is 200 mcg.

Other maintenance therapies can be found in the GINA guidelines and are provided at the approved or recommended doses. For example, in track 1, steps 1-2 budesonide-formoterol is given at a metered dose of 200/6 mcg (delivered 160/4.5 mcg) for patients 12 years and above. Track 1, steps 3-4 is aged 6-11 years budesonide-formoterol metered dose of 100/6 mcg (delivered 80/4.5 mcg), aged 12-17 years budesonide-formoterol metered dose of 200/6 mcg (delivered 160/4.5 mcg), aged 18 years and over budesonide-formoterol metered dose of 200/6 mcg (delivered 160/4.5 mcg) or beclomethasone-formoterol metered dose of 100/6 mcg (delivered 84.6/5.0 mcg). Track 1, step 5 is aged 12-17 years budesonide-formoterol metered dose of 200/6 mcg (delivered 160/4.5 mcg), aged 18 years and over budesonide-formoterol metered dose of 200/6 mcg (delivered 160/4.5 mcg) or beclomethasone-formoterol metered dose of 100/6 mcg (delivered 84.6/5.0 mcg).

The method of treatment of asthma of the present invention comprises PRN administration as rescue medication a fixed dose combination of fluticasone propionate and albuterol sulfate. The fluticasone propionate is typically administered at a delivered dose of 22-59 mcg per inhalation and the albuterol sulfate is administered at a delivered dose of 92-124 mcg per inhalation. Preferably, the fluticasone propionate is administered at a delivered dose of 23-56 mcg per inhalation and the albuterol sulfate is administered at a delivered dose of 97-119 mcg per inhalation. More preferably, the fluticasone propionate is administered at a delivered dose of 25-54 mcg per inhalation and the albuterol sulfate is administered at a delivered dose of 103-113 mcg per inhalation. In a particular embodiment, the fluticasone propionate is administered at a delivered dose of 26 or 51 mcg per inhalation and the albuterol sulfate is administered at a delivered dose of 108 mcg per inhalation.

In addition, the fluticasone propionate is typically administered at a metered dose of 26-63 mcg per inhalation and the albuterol sulfate is administered at a metered dose of 99-135 mcg per inhalation. Preferably, the fluticasone propionate is administered at a metered dose of 27-61 mcg per inhalation and the albuterol sulfate is administered at a metered dose of 105-129 mcg per inhalation. More preferably, the fluticasone propionate is administered at a metered dose of 29-58 mcg per inhalation and the albuterol sulfate is administered at a metered dose of 111-123 mcg per inhalation. In a particular embodiment, the fluticasone propionate is administered at a metered dose of 30 or 55 mcg per inhalation and the albuterol sulfate is administered at a metered dose of 117 mcg per inhalation.

Typically, the maximum daily metered dose does not exceed 6 doses, i.e. 12 inhalations (2 inhalations per dose).

The terms "metered dose" and "delivered dose" are conventional terms in the art, and have their standard meanings. That is, the metered dose is the mass of active that is available within the aerosol generator per actuation. This is the dose that is metered. The delivered dose is the mass of the active emitted per actuation that is actually available for inhalation at the mouth.

The method of treating asthma of the present invention in which the fixed-dose combination of fluticasone propionate and albuterol sulfate is used as reliever treatment reduces exacerbations, exposure to systemic corticosteroids, the number of asthma deteriorations, and improves symptoms for patients with asthma. While many patients are treated with ICS or ICS/LABA combinations as part of their maintenance therapy, many are noncompliant with this treatment. Multiple studies have shown that many of the asthma patients are noncompliant with their maintenance therapy. As patients' asthma deteriorates, they become symptomatic which prompts the use of more rescue medication. With the incorporation of ICS, in particular fluticasone propionate, and albuterol sulfate into a single inhaler, the patients gain relief of symptoms, but the combination will also treat the underlying inflammation that is responsible for the increase in symptoms and thus will reduce exacerbations compared to albuterol alone rescue treatment. Thus, the incorporation of ICS, in particular fluticasone propionate, and albuterol sulfate into a single inhaler minimizes the untoward effect of overuse of albuterol (or the single enantiomer, levalbuterol) administered as the sole rescue therapy (e.g. exacerbation, hospitalization and death).

Reducing the exposure to corticosteroid is also an important advantage to this approach. The short-term increase in ICS exposure is self-limited since the patient will either improve and use less rescue therapy, and thus less ICS, or the patient will present for treatment of a severe exacerbation, resulting in systemic corticosteroid treatment and higher exposure. In a sense, the patient self-titrates based on the requirement to treat asthma symptoms.

A fixed-dose dry powder inhalation composition of the present invention is used in the treatment of asthma, particularly in the treatment or prevention of bronchospasm in patients 4 years to <18 years old with reversible obstructive airway disease, and/or the prevention of exacerbations in patients 4 years to <18 years old, wherein the fixed dose dry powder inhalation composition is PRN administered as a rescue medication and wherein it comprises fluticasone propionate and albuterol sulfate. Thus, the present invention provides a method for treating asthma by treating or preventing bronchospasm in patients 4 years to <18 years old with reversible obstructive airways disease and/or by preventing exacerbations in patients 4 years to <18 years old comprising pro re nata (PRN) administration of a fixed-dose dry powder inhalation composition comprising fluticasone propionate and albuterol sulfate as a rescue medication.

In an embodiment of the present invention, the treatment is the prevention of bronchospasm in patients 4 years to <18 years old with reversible obstructive airway disease. In another embodiment, the treatment is the prevention of exacerbations of asthma in patients 4 years to <18 years old. In a preferred embodiment the patients 4 years to <18 years old includes either ≥4 to <12-year-old patients, ≥6 to <12-year-old patients, ≥12 to <18-year-old patients and combination thereof. In a more preferred embodiment, the ≥4 to <12-year-old patient age group includes at least one of 4-11 and 6-11 years old.

Specifically, the method/composition of the present invention provides one or more of prolonging the time to first severe clinical asthma exacerbation (CAE) when compared to albuterol sulfate rescue treatment, reducing the total annualized systemic corticosteroid (SCS) exposure when compared to albuterol sulfate rescue treatment, and reducing the annualized severe CAE rate when compared to albuterol sulfate rescue treatment. In an embodiment, the method/composition provides one or more of prolonging the time to first severe CAE when compared to albuterol sulfate rescue treatment, and reducing the total annualized SCS exposure when compared to albuterol sulfate rescue treatment. In another embodiment, the method/composition provides one or more of reducing the total annualized SCS exposure when compared to albuterol sulfate rescue treatment and reducing the annualized severe CAE rate when compared to albuterol sulfate rescue treatment. In another embodiment, the method/composition provides one or more of prolonging the time to first severe CAE when compared to albuterol sulfate rescue treatment, and reducing the annualized severe CAE rate when compared to albuterol sulfate rescue treatment.

In an embodiment, the method/composition of the present invention prolongs the time to first severe clinical asthma exacerbation (CAE) when compared to albuterol sulfate rescue treatment, and/or reduces the total annualized systemic corticosteroid (SCS) exposure when compared to albuterol sulfate rescue treatment, and/or reduces the annualized severe CAE rate when compared to albuterol sulfate rescue treatment.

In another embodiment, the method/composition of the present invention provides one or more of prolonging the time between severe CAEs when compared to albuterol sulfate rescue treatment and decreasing the amount of time a patient has a severe CAE when compared to albuterol sulfate rescue treatment.

In a preferred embodiment the method/composition of the present invention providing one or more of prolonging the time to first severe CAE when compared to albuterol sulfate rescue treatment, reducing the total annualized SCS exposure when compared to albuterol sulfate rescue treatment, and reducing the annualized severe clinical asthma exacerbation (CAE) rate when compared to albuterol sulfate rescue treatment also provides one or more of prolonging the time between severe CAEs when compared to albuterol sulfate rescue treatment, and decreasing the amount of time a patient has a severe CAE when compared to albuterol sulfate rescue treatment.

Albuterol sulfate rescue treatment as used herein refers to a PRN administration of albuterol sulfate only, wherein the patient can be also on a separate maintenance treatment of any kind of long-term asthma medication such as ICS, LABA, LAMA, SAMA, various biological medications, and combinations thereof. The preferred metered dose of albuterol sulfate is 117 mcg (and a 108 mcg delivered dose). If an ICS is used as maintenance treatment it may be administered as a monoproduct, or in combination with other long-term asthma drugs. The ICS can be the same as in the fixed-dose dry powder inhalation composition, i.e. fluticasone propionate, it can be another ICS, or a combination of ICS with any of the above listed actives as described hereinabove.

As used herein, the time to first severe CAE is calculated as the time from initiation of the treatment according to the present invention until the start date of the first severe CAE. An asthma exacerbation is considered severe if it results in at least one of the following.
- Treatment with SCS for at least 3 consecutive days to treat symptoms of asthma worsening. A single depo-injection of corticosteroids is considered equivalent to a 3-day course of oral corticosteroids.
- An urgent care or emergency room visit due to asthma that required SCS for at least 3 consecutive days.
- A hospitalization (defined as admission to an in-patient facility or observation unit) for ≥24 hours due to asthma.

Two severe CAEs must be separated by more than 7 days. If the end date of the first CAE and the start date of the second one is less than 7 days apart, then these are counted as one severe exacerbation.

In an alternative embodiment, the present invention provides also an improvement in asthma as defined by the Asthma Control Questionnaire-5 (ACQ-5) response at week 24, defined as achieving a decrease in score from baseline value of at least 0.5 when compared to albuterol sulfate rescue treatment, and/or an AQLQ+12/PAQLQ (patients aged ≥7 years) response at week 24, defined as achieving an increase in score from baseline of at least 0.5 when compared to albuterol sulfate rescue treatment. The improvement in asthma as presented in these questionnaires can be demonstrated for patients aged 6 years and older (<18). In a preferred embodiment the improvement can already be detected at week 12 for patients aged 6 <18 years old.

The present fixed-dose combination is the first ICS-SABA fixed-dose combination to be effective in the pediatric population. That is, in patients aged from ≥4 years to <18 years. In another embodiment, the present fixed-dose combination can be used to treat asthma patients aged from ≥6 years to <18 years. Pediatric patients are often subdivided into patients aged from ≥4 to <12 years and patients aged from ≥12 to <18 years. Sometimes, the older age group are referred to as adolescents.

The present invention may be applied to patients at any of the GINA steps 1-5, i.e. the patients are any one of GINA step 1-5 patients.

The fixed-dose dry powder inhalation composition of the present invention can be provided for example in two doses. One dose product is administered with an exemplified metered dose of 30 mcg fluticasone propionate and 117 mcg albuterol sulfate per inhalation (equivalent to 26 mcg and 108 mcg delivered dose per inhalation, respectively). The second dose product is administered with an exemplified metered dose of 55 mcg fluticasone propionate and 117 mcg albuterol sulfate per inhalation (equivalent to 51 mcg and 108 mcg delivered dose per inhalation, respectively). The amounts of albuterol sulfate set out herein, e.g. 117 mcg metered and 108 mcg delivered, are based on the total weight of the salt. These specific values correspond to 97.5 mcg and 90 mcg, respectively, based on the albuterol free base. Bioequivalent doses to the doses of the present invention are also included, particularly doses bioequivalent to the delivered doses, as described herein.

As mentioned before the FDC of budesonide and albuterol sulfate was found not to be effective in treating asthma patients aged from ≥4 years to <18 years, and it is therefore surprising that the present invention provides an effective treatment at fluticasone propionate doses of 26-63 mcg metered or 22-59 mcg delivered, e.g. at both exemplified fluticasone propionate doses of 30 mcg metered or 26 mcg delivered and 55 mcg metered or 51 mcg delivered.

The fixed-dose dry powder inhalation composition of the present invention is a dry powder formulation for inhalation. That means the product is provided with a powder carrier, such as lactose, particularly α-lactose monohydrate.

Such carriers are termed "coarse" carriers to distinguish them from fine particles which are entrained into the lung. They are well known in the art and are readily available commercially from a number of sources. A coarse carrier usually contains some fine particles of the same material (inherently present and/or deliberately added). Such fine particles assist with the release of the active ingredient(s) from the coarse carrier

In general, the particle size of the α-lactose monohydrate carrier should be such that it can be entrained in an air stream but not deposited in the key target sites of the lung. Accordingly, the α-lactose monohydrate preferably has the following particle size distribution as measured by laser diffraction: d10 is 20-40 µm; d50 is 55-65 µm; and d90 is 80-100 µm.

The lactose may contain inherent fine content (i.e. fine lactose) or they may also be deliberately added to the formulation. Such lactose has a particle size less than 10 µm in size, more likely 1-5 µm as measured by laser diffraction. In a preferred embodiment the % w/w of the particles that is smaller than 10 µm is less than 6 as measured by laser diffraction.

Dry powder inhalable formulations may also contain a ternary excipient. Ternary excipients are well-known in the art and are used for example to provide additional stability to the active ingredients. Typically, the additional stability is provided by reducing the amount of water adsorption and by promoting release of the active ingredient from the coarse carrier particles.

Ternary excipients are also known as force control agents, lubricants or anti-adherents. They use the term "ternary" because they add a third material to the formulation over the active ingredient(s) and the carrier. It should be noted that the coarse carrier (i.e. α-lactose monohydrate) usually contains some fine particles of the same material (inherently present and/or deliberately added). Such fine particles composed of the same material as the coarse carrier are not ternary excipients.

Ternary excipients are also known as force control agents, lubricants or anti-adherents. They use the term "ternary" because they add a third material to the formulation over the active ingredient(s) and the carrier. It should be noted that the coarse carrier (i.e. α-lactose monohydrate) usually contains some fine particles of the same material (inherently present and/or deliberately added). Such fine particles composed of the same material as the coarse carrier are not ternary excipients.

In one embodiment, the dry powder inhalable formulation of the present invention does not include a ternary excipient. For example, the formulation may consist of fluticasone propionate, albuterol sulfate and lactose (e.g. an α-lactose monohydrate carrier, optionally containing fine α-lactose monohydrate particles).

In another embodiment, the dry powder inhalable formulation of the present invention further comprises a ternary excipient.

Typical examples of ternary excipients which may be formulated within the formulation of the present invention include metal stearates (such as magnesium and calcium stearate), fatty acids (e.g. stearic acid), amino acids (such as leucine) and phospholipids (such as lecithin).

It is preferred wherein the ternary excipient formulated within the formulation of the present invention is magnesium stearate. It is also preferred wherein the proportion of magnesium stearate contained within the formulation is 0.01-3.0% w/w by weight of the formulation. Ternary excipients can be used to provide additional stability.

The fixed dose dry powder composition of the present invention is preferably prepared by mixing fluticasone propionate, albuterol sulfate and lactose. In the formulation of the present invention 99% w/w of the particles of fluticasone propionate and of albuterol sulfate each are less than 10 µm as measured by laser diffraction, preferably 90% w/w of the fluticasone propionate particles are less than 6 µm as measured by laser diffraction. And 90% w/w of the albuterol sulfate particles are smaller than 5 µm as measured by laser diffraction.

The particle sizes (mass median aerodynamic diameter, MMAD) of the fluticasone propionate used within the process of the present invention are less than 10 µm in size, more preferably 1-4 µm, and most preferably less than 3 µm. MMAD may be measured using a next generation impactor (NGI).

The particle sizes (mass median aerodynamic diameter, MMAD) of the albuterol sulfate used within the process of the present invention are less than 10 µm in size, more preferably 1-4 µm and most preferably less than 2 µm. MMAD may be measured using a next generation impactor (NGI).

This particle size ensures that the particles effectively adhere to the carrier during mixing, and also that the particles disperse and become entrained in the air stream and deposited in the lower lung (i.e. upon actuation of an inhaler device).

The dry powder formulation may be metered and filled into capsules, e.g. gelatin or hydroxypropyl methylcellulose capsules, such that the capsule contains a unit dose of active ingredient. When the dry powder is in a capsule containing a unit dose of active ingredient, the total amount of composition will depend on the size of the capsules and the characteristics of the inhalation device with which the capsules are being used. However, typical examples of total fill weights of dry powder per capsule are 1-25 mg. The formulation may also be filled into blister strips. Alternatively, the dry powder composition according to the invention may be filled into the reservoir of a multi-dose dry powder inhaler (MDPI).

Preferably, the multi-dose dry powder inhaler includes a cyclone deagglomerator for breaking up agglomerates of the active ingredients and carrier. This occurs prior to inhalation of the powder by a patient. The deagglomerator includes an inner wall defining a swirl chamber extending along an axis from a first end to a second end, a dry powder supply port, two inlet ports, and an outlet port.

The supply port is in the first end of the swirl chamber for providing fluid communication between a dry powder delivery passageway of the inhaler and the first end of the swirl chamber. The inlet port is in the inner wall of the swirl chamber adjacent to the first end of the swirl chamber and provides fluid communication between a region exterior to the deagglomerator and the swirl chamber. The outlet port provides fluid communication between the second end of the swirl chamber and a region exterior to the deagglomerator.

A breath induced low pressure at the outlet port causes air flows into the swirl chamber through the dry powder supply port and the inlet port. The air flows collide with each other and with the wall of the swirl chamber prior to exiting through the outlet port, such that the active is detached from the carrier (lactose). The deagglomerator further includes vanes at the first end of the swirl chamber for creating additional collisions and impacts of entrained powder.

A first breath-actuated air flow is directed for entraining a dry powder from an inhaler into a first end of a chamber extending longitudinally between the first end and a second end, the first air flow directed in a longitudinal direction.

A second breath-actuated airflow is directed in a substantially transverse direction into the first end of the chamber such that the air flows collide and substantially combine.

Then, a portion of the combined air flows is deflected in a substantially longitudinal direction towards a second end of the chamber, and a remaining portion of the combined air flows is directed in a spiral path towards the second end of the chamber. All the combined air flows and any dry powder entrained therein are then delivered from the second end of the chamber to a patient's mouth.

The deagglomerator ensures that particles of the actives are small enough for adequate penetration of the powder into a bronchial region of a patient's lungs during inhalation by the patient.

Thus, preferably, where the dry powder formulation of the present invention is used in conjunction with a multi-dose dry powder inhaler device, the deagglomerator of the inhaler device comprises: an inner wall defining a swirl chamber extending along an axis from a first end to a second end; a dry powder supply port in the first end of the swirl chamber for providing fluid communication between a dry powder delivery passageway of the inhaler and the first end of the swirl chamber; at least one inlet port in the inner wall of the swirl chamber adjacent to the first end of the swirl chamber providing fluid communication between a region exterior to the deagglomerator and the first end of the swirl chamber; an outlet port providing fluid communication between the second end of the swirl chamber and a region exterior to the deagglomerator; and vanes at the first end of the swirl chamber extending at least in part radially outwardly from the axis of the chamber, each of the vanes having an oblique surface facing at least in part in a direction transverse to the axis; whereby a breath induced low pressure at the outlet port causes air flows into the swirl chamber through the dry powder supply port and the inlet port.

In a preferred embodiment, the flow rate of the inhaler at a 4 kPa pressure drop is 59-71 L/min, most preferably 63 L/min. The flow resistance is preferably 0.028-0.034 KPa^{0.5}/L/min.

The inhaler preferably has a reservoir for containing the formulation and an arrangement for delivering a metered dose of the formulation from the reservoir. The reservoir is typically a pressure system. The inhaler preferably includes: a sealed reservoir including a dispensing port; a channel communicating with the dispensing port and including a pressure relief port; a conduit providing fluid communication between an interior of the sealed reservoir and the pressure relief port of the channel; and a cup assembly movably received in the channel and including, a recess adapted to receive formulation when aligned with the dispensing port, a first sealing surface adapted to seal the dispensing port when the recess is unaligned with the dispensing port, and a second sealing surface adapted to sealing the pressure relief port when the recess is aligned with the dispensing port and unseal the pressure relief port when the recess is unaligned with the dispensing port.

The inhaler preferably has a dose counter. The inhaler includes a mouthpiece for patient inhalation, a dose-metering arrangement including a pawl movable along a predetermined path during the metering of a dose of formulation to the mouthpiece by the dose-metering arrangement, and a dose counter.

In a preferred form, the dose counter includes a bobbin, a rotatable spool, and a rolled ribbon received on the bobbin, rotatable about an axis of the bobbin. The ribbon has indicia thereon successively extending between a first end of the ribbon secured to the spool and a second end of the ribbon positioned on the bobbin. The dose counter also includes teeth extending radially outwardly from the spool into the predetermined path of the pawl so that the spool is rotated by the pawl and the ribbon advanced onto the spool during the metering of a dose to the mouthpiece.

The preferred inhaler includes a simple, accurate and consistent mechanical dose metering system that dispenses dry powdered formulation in discrete amounts or doses for patient inhalation, a reservoir pressure system that ensures consistently dispensed doses, and a dose counter indicating the number of doses remaining in the inhaler.

The inhaler used in the present invention may also have electronic connectivity. This provides an inhalation monitoring system comprising: an inhaler comprising a medicament delivery apparatus configured to deliver the fixed-dose product of the present invention to a user during an inhalation of the user; an inhalation monitoring apparatus configured to, during said inhalation, gather data for determining a measure of the user's lung function and/or lung health and/or inhalation technique; and a processor configured to receive said data from said inhalation monitoring apparatus and, using the data, determine a measure of the user's lung function and/or lung health and/or inhalation technique. Further details may be found in WO 2016/090260 and WO 2020/222146.

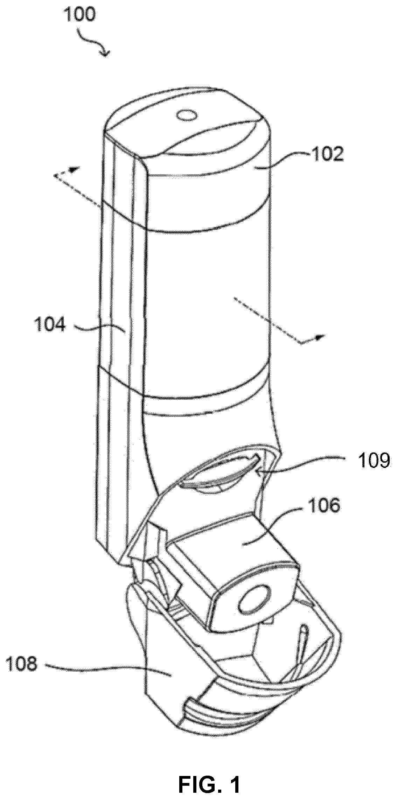

Figs. 1-4 provide views of an inhaler 100 for administering a dry powder inhalation composition according to any of the embodiments described herein.

The inhaler 100 may include a top cap 102, a main housing 104, a mouthpiece 106, a mouthpiece cover 108, an air vent 109, and an electronics module 120. The mouthpiece cover 108 may be hinged to the main housing 104 so that it may open and close to expose the mouthpiece 106. Although illustrated as a hinged connection, the mouthpiece cover 106 may be connected to the inhaler 100 through other types of connections. Moreover, while the electronics module 120 is illustrated as housed within the top cap 102 at the top of the main housing 104, the electronics module 120 may be integrated and/or housed within the main body 104 of the inhaler 100.

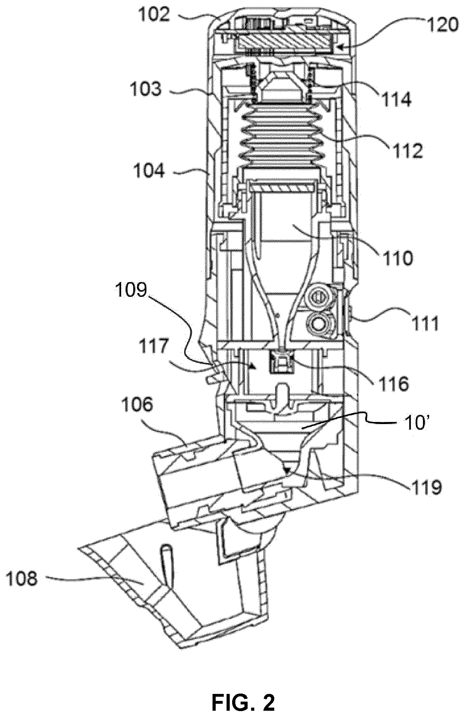

Fig. 2 provides a cross-sectional interior perspective view of the example inhaler 100. Inside the main housing 104, the inhaler 100 may include a medication reservoir 110 (e.g. a hopper), a bellows 112, a bellows spring 114, a yoke (not visible), a dosing cup 116, a dosing chamber 117, a deagglomerator 10', and a flow pathway 119. The medication reservoir 110 may include medication, in particular the dry powder inhalation composition, for delivery to the subject. When the mouthpiece cover 108 is moved from the closed to the open position, the bellows 112 may compress to deliver a dose of medication from the medication reservoir 110 to the dosing cup 116. Thereafter, a subject may inhale through the mouthpiece 106 in an effort to receive the dose of medication.

In a preferred embodiment, the dosing cup 116 has a capacity of 5-10 mm³, and more preferably is a size 3 dose cup 116, which corresponds to a capacity of 7 mm³.

The airflow generated from the subject's inhalation may cause the deagglomerator 10' to aerosolize the dose of medication by breaking down the agglomerates of the medicament in the dosing cup 116. The deagglomerator 10' may be configured to aerosolize the medication when the airflow through the flow pathway 119 meets or exceeds a particular rate, or is within a specific range. When aerosolized, the dose of medication may travel from the dosing cup 116, into the dosing chamber 117, through the flow pathway 119, and out of the mouthpiece 106 to the subject. If the airflow through the flow pathway 119 does not meet or exceed a particular rate, or is not within a specific range, the medication may remain in the dosing cup 116. In the event that the medication in the dosing cup 116 has not been aerosolized by the deagglomerator 10', another dose of medication may not be delivered from the medication reservoir 110 when the mouthpiece cover 108 is subsequently opened. Thus, a single dose of medication may remain in the dosing cup until the dose has been aerosolized by the deagglomerator 10'.

As the subject inhales through the mouthpiece 106, air may enter the air vent 109 to provide a flow of air for delivery of the medication to the subject. The flow pathway 119 may extend from the dosing chamber 117 to the end of the mouthpiece 106, and include the dosing chamber 117 and the internal portions of the mouthpiece 106. The dosing cup 116 may reside within or adjacent to the dosing chamber 117. Further, the inhaler 100 may include a dose counter 111 that is configured to be initially set to a number of total doses of medication within the medication reservoir 110 and to decrease by one each time the mouthpiece cover 108 is moved from the open position to the closed position.

When a dose of medication is delivered, a dose confirmation may be stored in a memory included in the electronics module 120 as dose confirmation information.

The top cap 102 may be attached to the main housing 104. For example, the top cap 102 may be attached to the main housing 104 through the use of one or more clips that engage recesses on the main housing 104. The top cap 102 may overlap a portion of the main housing 104 when connected, for example, such that a substantially pneumatic seal 103 exists between the top cap 102 and the main housing 104.

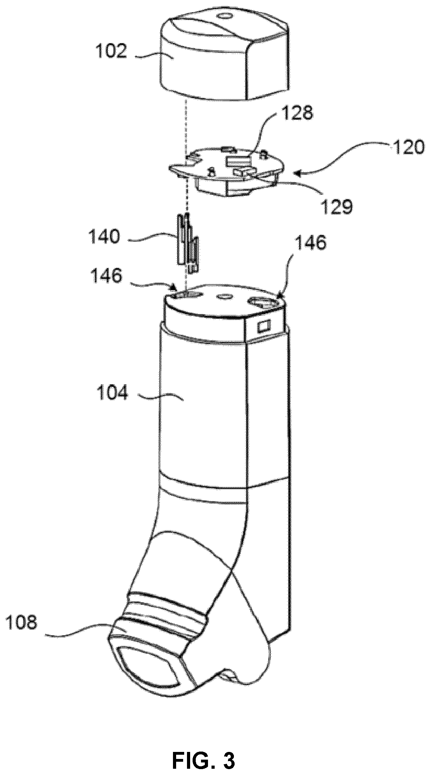

Fig. 3 is an exploded perspective view of the example inhaler 100 with the top cap 102 removed to expose the electronics module 120. As shown in Fig. 3, the top surface of the main housing 104 may include one or more (e.g. two) orifices 146. One or more (e.g. both) of the orifices 146 may be configured to accept a slider 140. For example, when the top cap 102 is attached to the main housing 104, the slider 140 may protrude through the top surface of the main housing 104 via one of the orifices 146.

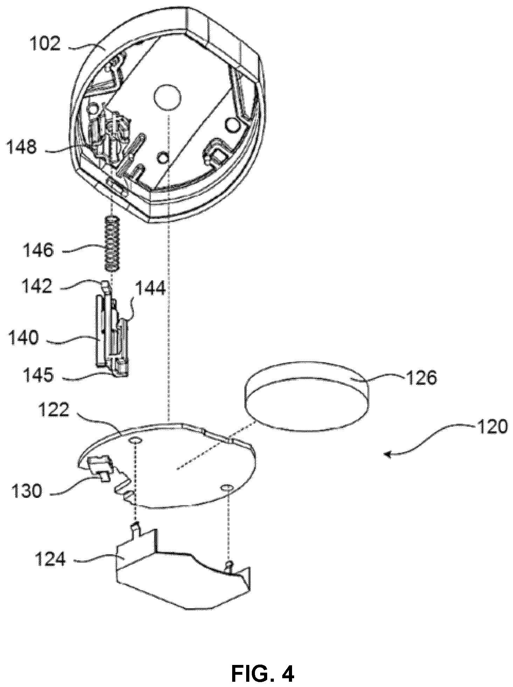

Fig. 4 is an exploded perspective view of the top cap 102 and the electronics module 120 of the example inhaler 100. As shown in Fig. 4, the slider 140 may define an arm 142, a stopper 144, and a distal end 145. The distal end 145 may be a bottom portion of the slider 140. The distal end 145 of the slider 140 may be configured to abut the yoke that resides within the main housing 104 (e.g. when the mouthpiece cover 108 is in the closed or partially open position). The distal end 145 may be configured to abut a top surface of the yoke when the yoke is in any radial orientation. For example, the top surface of the yoke may include a plurality of apertures (not shown), and the distal end 145 of the slider 140 may be configured to abut the top surface of the yoke, for example, whether or not one of the apertures is in alignment with the slider 140.

The top cap 102 may include a slider guide 148 that is configured to receive a slider spring 146 and the slider 140. The slider spring 146 may reside within the slider guide 148. The slider spring 146 may engage an inner surface of the top cap 102, and the slider spring 146 may engage (e.g. abut) an upper portion (e.g. a proximate end) of the slider 140. When the slider 140 is installed within the slider guide 148, the slider spring 146 may be partially compressed between the top of the slider 140 and the inner surface of the top cap 102. For example, the slider spring 146 may be configured such that the distal end 145 of the slider 140 remains in contact with the yoke when the mouthpiece cover 108 is closed. The distal end 145 of the slider 145 may also remain in contact with the yoke while the mouthpiece cover 108 is being opened or closed. The stopper 144 of the slider 140 may engage a stopper of the slider guide 148, for example, such that the slider 140 is retained within the slider guide 148 through the opening and closing of the mouthpiece cover 108, and vice versa. The stopper 144 and the slider guide 148 may be configured to limit the vertical (e.g. axial) travel of the slider 140. This limit may be less than the vertical travel of the yoke. Thus, as the mouthpiece cover 108 is moved to a fully open position, the yoke may continue to move in a vertical direction towards the mouthpiece 106 but the stopper 144 may stop the vertical travel of the slider 140 such that the distal end 145 of the slider 140 may no longer be in contact with the yoke.

More generally, the yoke may be mechanically connected to the mouthpiece cover 108 and configured to move to compress the bellows spring 114 as the mouthpiece cover 108 is opened from the closed position and then release the compressed bellows spring 114 when the mouthpiece cover reaches the fully open position, thereby causing the bellows 112 to deliver the dose from the medication reservoir 110 to the dosing cup 116. The yoke may be in contact with the slider 140 when the mouthpiece cover 108 is in the closed position. The slider 140 may be arranged to be moved by the yoke as the mouthpiece cover 108 is opened from the closed position and separated from the yoke when the mouthpiece cover 108 reaches the fully open position.

The movement of the slider 140 during the dose metering may cause the slider 140 to engage and actuate a switch 130. The switch 130 may trigger the electronics module 120 to register the dose metering. The slider 140 may be regarded in this example as the means by which a use determination system is configured to register the metering of the dose by the dose metering system, each metering being thereby indicative of an inhalation performed by the subject using the inhaler 100.

Actuation of the switch 130 by the slider 140 may also, for example, cause the electronics module 120 to transition from the first power state to a second power state, and to sense an inhalation by the subject from the mouthpiece 106.

The electronics module 120 may include a printed circuit board (PCB) assembly 122, a switch 130, a power supply (e.g. a battery 126), and/or a battery holder 124. Referring to Figs. 3 and 4, the PCB assembly 122 may include surface mounted components, such as a sensor system 128, a wireless communication circuit 129, the switch 130, and/or one or more indicators (not shown), such as one or more light emitting diodes (LEDs). The electronics module 120 may include a controller (e.g. a processor) and/or memory. The controller and/or memory may be physically distinct components of the PCB assembly 122. Alternatively, the controller and memory may be part of another chipset mounted on the PCB assembly 122, for example, the wireless communication circuit 129 may include the controller and/or memory for the electronics module 120. The controller of the electronics module 120 may include a microcontroller, a programmable logic device (PLD), a microprocessor, an application specific integrated circuit (ASIC), a field programmable gate array (FPGA), or any suitable processing device or control circuit.

The controller may access information from, and store data in the memory. The memory may include any type of suitable memory, such as non-removable memory and/or removable memory. The non-removable memory may include random-access memory (RAM), read-only memory (ROM), or any other type of memory storage device. The removable memory may include a subscriber identity module (SIM) card, a memory stick, a secure digital (SD) memory card, and the like. The memory may be internal to the controller. The controller may also access data from, and store data in, memory that is not physically located within the electronics module 120, such as on a server or a smart phone.

The sensor system 128 may include one or more sensors. The sensor system 128 may include one or more sensors, for example, of different types, such as, but not limited to one or more pressure sensors, temperature sensors, humidity sensors, orientation sensors, acoustic sensors, and/or optical sensors. The one or more pressure sensors may include a barometric pressure sensor (e.g. an atmospheric pressure sensor), a differential pressure sensor, an absolute pressure sensor, and/or the like. The sensors may employ microelectromechanical systems (MEMS) and/or nanoelectromechanical systems (NEMS) technology. The sensor system 128 may be configured to provide an instantaneous reading (e.g. pressure reading) to the controller of the electronics module 120 and/or aggregated readings (e.g. pressure readings) overtime. As illustrated in Figs. 2 and 3, the sensor system 128 may reside outside the flow pathway 119 of the inhaler 100, but may be pneumatically coupled to the flow pathway 119.

The controller of the electronics module 120 may receive signals corresponding to measurements from the sensor system 128. The controller may calculate or determine one or more airflow metrics using the signals received from the sensor system 128. The airflow metrics may be indicative of a profile of airflow through the flow pathway 119 of the inhaler 100. For example, if the sensor system 128 records a change in pressure of 0.3 kilopascals (kPa), the electronics module 120 may determine that the change corresponds to an airflow rate of approximately 45 liters per minute (Lpm) through the flow pathway 119.

The pressure measurement readings and/or the computed airflow metrics may be indicative of the quality or strength of inhalation from the inhaler 100. For example, when compared to a particular threshold or range of values, the readings and/or metrics may be used to categorize the inhalation as a certain type of event, such as a good inhalation event, a low inhalation event, a no inhalation event, or an excessive inhalation event. The categorization of the inhalation may be usage parameters stored as personalized data of the subject.

The no inhalation event may be associated with pressure measurement readings and/or airflow metrics below a particular threshold, such as an airflow rate less than 30 Lpm. The no inhalation event may occur when a subject does not inhale from the mouthpiece 106 after opening the mouthpiece cover 108 and during the measurement cycle. The no inhalation event may also occur when the subject's inspiratory effort is insufficient to ensure proper delivery of the medication via the flow pathway 119, such as when the inspiratory effort generates insufficient airflow to activate the deagglomerator 10' and, thus, aerosolize the medication in the dosing cup 116.

The low inhalation event may be associated with pressure measurement readings and/or airflow metrics within a particular range, such as an airflow rate between 30 Lpm and 45 Lpm. The low inhalation event may occur when the subject inhales from the mouthpiece 106 after opening the mouthpiece cover 108 and the subject's inspiratory effort causes at least a partial dose of the medication to be delivered via the flow pathway 119. That is, the inhalation may be sufficient to activate the deagglomerator 10' such that at least a portion of the medication is aerosolized from the dosing cup 116.

The good inhalation event may be associated with pressure measurement readings and/or airflow metrics above the low inhalation event, such as an airflow rate between 45 Lpm and 200 Lpm. The good inhalation event may occur when the subject inhales from the mouthpiece 106 after opening the mouthpiece cover 108 and the subject's inspiratory effort is sufficient to ensure proper delivery of the medication via the flow pathway 119, such as when the inspiratory effort generates sufficient airflow to activate the deagglomerator 10' and aerosolize a full dose of medication in the dosing cup 116.

The excessive inhalation event may be associated with pressure measurement readings and/or airflow metrics above the good inhalation event, such as an airflow rate above 200 Lpm. The excessive inhalation event may occur when the subject's inspiratory effort exceeds the normal operational parameters of the inhaler 100. The excessive inhalation event may also occur if the inhaler 100 is not properly positioned or held during use, even if the subject's inspiratory effort is within a normal range. For example, the computed airflow rate may exceed 200 Lpm if the air vent 109 is blocked or obstructed (e.g. by a finger or thumb) while the subject is inhaling from the mouthpiece 106.

Any suitable thresholds or ranges may be used to categorize a particular event. Some or all of the events may be used. For example, the no inhalation event may be associated with an airflow rate below 45 Lpm and the good inhalation event may be associated with an airflow rate between 45 Lpm and 200 Lpm. As such, the low inhalation event may not be used at all in some cases.

The pressure measurement readings and/or the computed airflow metrics may also be indicative of the direction of flow through the flow pathway 119 of the inhaler 100. For example, if the pressure measurement readings reflect a negative change in pressure, the readings may be indicative of air flowing out of the mouthpiece 106 via the flow pathway 119. If the pressure measurement readings reflect a positive change in pressure, the readings may be indicative of air flowing into the mouthpiece 106 via the flow pathway 119. Accordingly, the pressure measurement readings and/or airflow metrics may be used to determine whether a subject is exhaling into the mouthpiece 106, which may signal that the subject is not using the inhaler 100 properly.

The personalized data collected from, or calculated based on, the usage of the inhaler 100 (e.g. pressure metrics, airflow metrics, lung function metrics, dose confirmation information, etc.) may be computed and/or assessed via external devices as well (e.g. partially or entirely). More specifically, the wireless communication circuit 129 in the electronics module 120 may include a transmitter and/or receiver (e.g. a transceiver), as well as additional circuity. For example, the wireless communication circuit 129 may include a Bluetooth chip set (e.g. a Bluetooth Low Energy chip set), a ZigBee chipset, a Thread chipset, etc. As such, the electronics module 120 may wirelessly provide the personalized data, such as pressure measurements, airflow metrics, lung function metrics, dose confirmation information, and/or other conditions related to usage of the inhaler 100, to an external device, including a smart phone. The personalized data may be provided in real time to the external device to enable an assessment of a status of the subject's respiratory disease to be generated based on real-time data from the inhaler 100 that indicates time of use, how the inhaler 100 is being used, and personalized data about the user of the inhaler, such as real-time data related to the subject's lung function and/or medical treatment. The external device may include software for processing the received information and for providing compliance and adherence feedback to users of the inhaler 100 via a graphical user interface (GUI).

The airflow metrics may include personalized data that is collected from the inhaler 100 in real-time, such as one or more of an average flow of an inhalation/exhalation, a peak flow of an inhalation/exhalation (e.g. a maximum inhalation received), a volume of an inhalation/exhalation, a time to peak of an inhalation/exhalation, and/or the duration of an inhalation/exhalation. The airflow metrics may also be indicative of the direction of flow through the flow pathway 119. That is, a negative change in pressure may correspond to an inhalation from the mouthpiece 106, while a positive change in pressure may correspond to an exhalation into the mouthpiece 106. When calculating the airflow metrics, the electronics module 120 may be configured to eliminate or minimize any distortions caused by environmental conditions. For example, the electronics module 120 may re-zero to account for changes in atmospheric pressure before or after calculating the airflow metrics. The one or more pressure measurements and/or airflow metrics may be timestamped and stored in the memory of the electronics module 120.

In addition to the airflow metrics, the inhaler 100, or another computing device, may use the airflow metrics to generate additional personalized data. For example, the controller of the electronics module 120 of the inhaler 100 may translate the airflow metrics into other metrics that indicate the subject's lung function and/or lung health that are understood to medical practitioners, such as peak inspiratory flow metrics, peak expiratory flow metrics, and/or forced expiratory volume in 1 second (FEV1), for example. The electronics module 120 of the inhaler may determine a measure of the subject's lung function and/or lung health using a mathematical model such as a regression model. The mathematical model may identify a correlation between the total volume of an inhalation and FEV1. The mathematical model may identify a correlation between peak inspiratory flow and FEV1. The mathematical model may identify a correlation between the total volume of an inhalation and peak expiratory flow. The mathematical model may identify a correlation between peak inspiratory flow and peak expiratory flow.

The battery 126 may provide power to the components of the PCB assembly 122. The battery 126 may be any suitable source for powering the electronics module 120, such as a coin cell battery, for example. The battery 126 may be rechargeable or non-rechargeable. The battery 126 may be housed by the battery holder 124. The battery holder 124 may be secured to the PCB assembly 122 such that the battery 126 maintains continuous contact with the PCB assembly 122 and/or is in electrical connection with the components of the PCB assembly 122. The battery 126 may have a particular battery capacity that may affect the life of the battery 126. As will be further discussed below, the distribution of power from the battery 126 to the one or more components of the PCB assembly 122 may be managed to ensure the battery 126 can power the electronics module 120 over the useful life of the inhaler 100 and/or the medication contained therein.

In a connected state, the communication circuit and memory may be powered on and the electronics module 120 may be "paired" with an external device, such as a smart phone. The controller may retrieve data from the memory and wirelessly transmit the data to the external device. The controller may retrieve and transmit the data currently stored in the memory. The controller may also retrieve and transmit a portion of the data currently stored in the memory. For example, the controller may be able to determine which portions have already been transmitted to the external device and then transmit the portion(s) that have not been previously transmitted. Alternatively, the external device may request specific data from the controller, such as any data that has been collected by the electronics module 120 after a particular time or after the last transmission to the external device. The controller may retrieve the specific data, if any, from the memory and transmit the specific data to the external device.

The data stored in the memory of the electronics module 120 (e.g. the signals generated by the switch 130, the pressure measurement readings taken by the sensory system 128 and/or the airflow metrics computed by the controller of the PCB assembly 122) may be transmitted to an external device, which may process and analyze the data to determine usage parameters associated with the inhaler 100. Further, a mobile application residing on the mobile device may generate feedback for the user based on data received from the electronics module 120. For example, the mobile application may generate daily, weekly, or monthly report, provide confirmation of error events or notifications, provide instructive feedback to the subject, and/or the like.

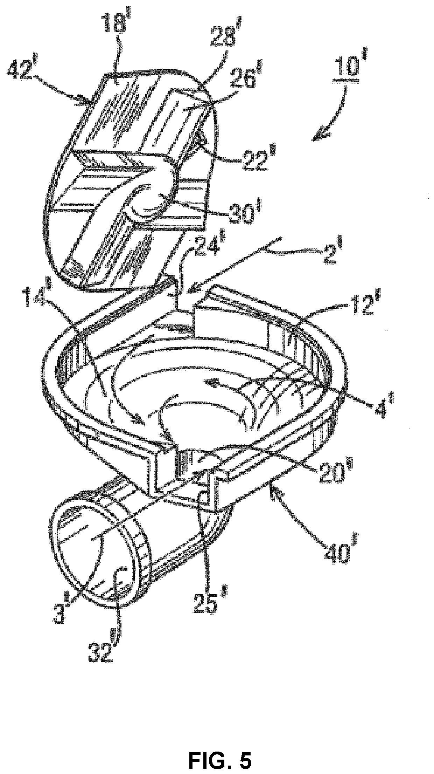

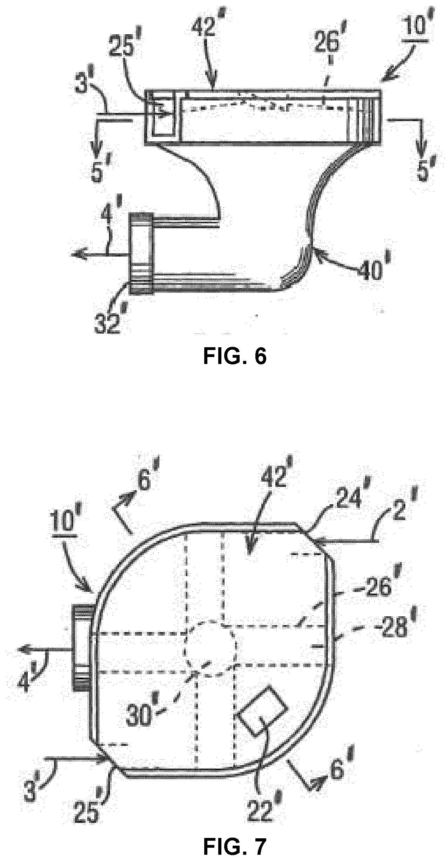

Referring to Figs. 5-10, the deagglomerator 10' breaks down agglomerates of dry powder formulation, or formulation and carrier, before the dry powder leaves the inhaler 100 through the mouthpiece 106. Thus, the agglomerates of dry powder formulation, or formulation and carrier, are broken down by the deagglomerator 10' before inhalation of the formulation by a patient.

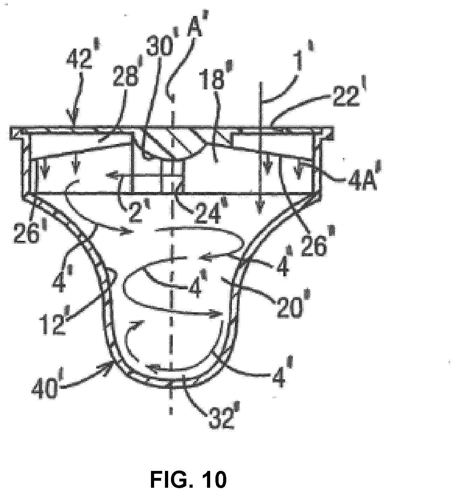

In general, the deagglomerator 10' includes an inner wall 12' defining a swirl chamber 14' extending along an axis A' (see Fig. 10) from a first end 18' to a second end 20'. The swirl chamber 14' includes circular cross-sectional areas arranged transverse to the axis A', that decrease from the first end 18' to the second end 20' of the swirl chamber 14', such that any air flow traveling from the first end of the swirl chamber to the second end is constricted and at least in part collide with the inner wall 12' of the chamber.

Preferably, the cross-sectional areas of the swirl chamber 14' decrease monotonically. In addition, the inner wall 12' is preferably convex, i.e. arches inwardly towards the axis A', as shown best in Fig. 10.

As shown in Figs. 5, 7 and 10, the deagglomerator 10' also includes a dry powder supply port 22' in the first end 18' of the swirl chamber 14' for providing fluid communication between a dry powder delivery passageway of an inhaler and the first end 18' of the swirl chamber 14'. Preferably, the dry powder supply port 22' faces in a direction substantially parallel with the axis A' such that an air flow, illustrated by arrow 1' in Fig. 10, entering the chamber 14' through the supply port 22' is at least initially directed parallel with respect to the axis A' of the chamber.

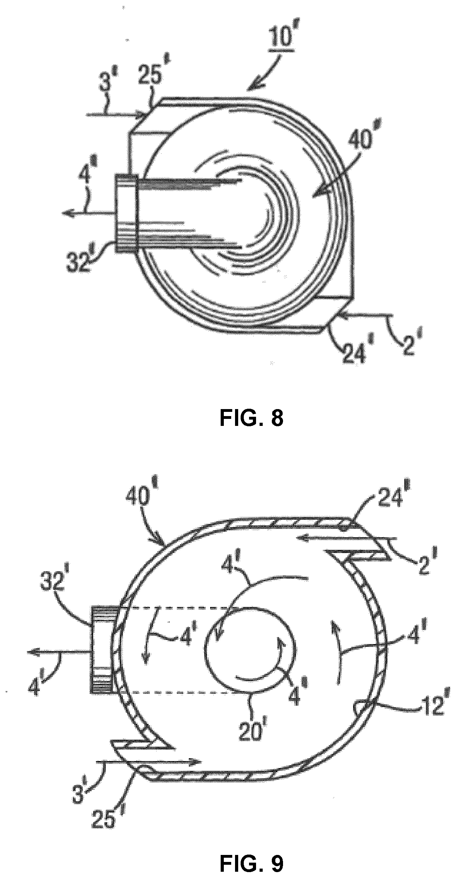

Referring to Figs. 5-10, the deagglomerator 10' additionally includes at least one inlet port 24' in the inner wall 12' of the swirl chamber 14' adjacent to or near the first end 18' of the chamber providing fluid communication between a region exterior to the deagglomerator and the first end 18' of the swirl chamber 14'. Preferably, the at least one inlet port comprises two diametrically opposed inlet ports 24', 25' that extend in a direction substantially transverse to the axis A' and substantially tangential to the circular cross-section of the swirl chamber 14'. As a result, air flows, illustrated by arrows 2' and 3' in Figs. 5 and 9, entering the chamber 14' through the inlet ports are at least initially directed transverse with respect to the axis A' of the chamber and collide with the air flow 1' entering through the supply port 22' to create turbulence. The combined air flows, illustrated by arrow 4' in Figs. 9 and 10, then collide with the inner wall 12' of the chamber 14', form a vortex, and create additional turbulence as they move towards the second end 20' of the chamber.

Referring to Figs. 5-7 and 10, the deagglomerator 10' includes vanes 26' at the first end 18' of the swirl chamber 14' extending at least in part radially outwardly from the axis A' of the chamber. Each of the vanes 26' has an oblique surface 28' facing at least in part in a direction transverse to the axis A' of the chamber. The vanes 26' are sized such that at least a portion 4A' of the combined air flows 4' collide with the oblique surfaces 28', as shown in Fig. 10. Preferably, the vanes comprise four vanes 26', each extending between a hub 30' aligned with the axis A' and the wall 12' of the swirl chamber 14'.

As shown in Figs. 5-10, the deagglomerator 10' further includes an outlet port 32' providing fluid communication between the second end 20' of the swirl chamber 14' and a region exterior to the deagglomerator. A breath induced low pressure at the outlet port 32' causes the air flow 1' through the supply port 22' and the air flows 2',3' through the inlet ports and draws the combined air flow 4' through the swirl chamber 14'. The combined air flow 4' then exits the deagglomerator through the outlet port 32'. Preferably the outlet port 32' extends substantially transverse to the axis A', such that the air flow 4' will collide with an inner wall of the outlet port 32' and create further turbulence. The term "substantially transverse" is intended to cover the outlet port 32' extending perpendicularly, in other words at 90 degrees, with respect to the axis A', but also the outlet port 32' extending at other angles with respect to the axis A', provided that the above-mentioned further turbulence can be created via collision of the air flow 4' with the outlet port's 32' inner wall. For example, the outlet port 32' may extend at an angle of about 15 degrees with respect to a perpendicular of the axis A'.

During use of the deagglomerator 10' in combination with the inhaler, patient inhalation at the outlet port 32' causes air flows 1',2',3' to enter through, respectively, the dry powder supply port 22' and the inlet ports. Although not shown, the air flow 1' through the supply port 22' entrains the dry powder into the swirl chamber 14'. The air flow 1' and entrained dry powder are directed by the supply port 22' into the chamber in a longitudinal direction, while the air flows 2',3' from the inlet ports are directed in a transverse direction, such that the air flows collide and substantial combine.

A portion of the combined air flow 4' and the entrained dry powder then collide with the oblique surfaces 28' of the vanes 26' causing particles and any agglomerates of the dry powder to impact against the oblique surfaces and collide with each other. The geometry of the swirl chamber 14' causes the combined air flow 4' and the entrained dry powder to follow a turbulent, spiral path, or vortex, through the chamber. As will be appreciated, the decreasing cross-sections of the swirl chamber 14' continuously changes the direction and increases the velocity of the spiraling combined air flow 4' and entrained dry powder. Thus, particles and any agglomerates of the dry powder constantly impact against the wall 12' of the swirl chamber 14' and collide with each other, resulting in a mutual grinding or shattering action between the particles and agglomerates. In addition, particles and agglomerates deflected off the oblique surfaces 28' of the vanes 26' cause further impacts and collisions.

Upon exiting the swirl chamber 14', the direction of the combined air flow 4 and the entrained dry powder is again changed to a transverse direction with respect to the axis A', through the outlet port 32'. The combined air flow 4' and the entrained dry powder retain a swirl component of the flow, such that the air flow 4' and the entrained dry powder spirally swirls through the outlet port 32'. The swirling flow causes additional impacts in the outlet port 32' so as to result in further breaking up of any remaining agglomerates prior to being inhaled by a patient.

As shown in Figs. 5-10, the deagglomerator is preferably assembled from two pieces: a cup-like base 40' and a cover 42'. The base 40' and the cover 42' are connected to form the swirl chamber 14'. The cup-like base 40' includes the wall 12' and the second end 20' of the chamber and defines the outlet port 32'. The base 40' also includes the inlet ports of the swirl chamber 14'. The cover 42' forms the vanes 26' and defines the supply port 22'.

The base 40' and the cover 42' of the deagglomerator are preferably manufactured from a plastic such as ABS for the base and polypropylene for the cover, but may be manufactured from metal or another suitable material. The base 40' and the cover 42' can be connected to each other in any suitable manner, for example by the base 40' being ultrasonically welded to the spacer 38', and the cover 42' being push-fitted into the underside of the spacer 38' and held in place.

The inhaler 100 shown in Figs. 1-4 may include the particular deagglomerator 10' described with reference to Figs. 5-10. However, the inhaler 100 is not limited to use with the deagglomerator shown in Figs. 1-4 and can be used with other types of deagglomerators or a simple swirl chamber.

It is also noted that the inhaler 100 having electronic connectivity is not intended to be limiting. In this respect, the present disclosure contemplates the inhaler 100 having the basic mechanical structure described above with reference to Figs. 1-10, or the mechanical structure described in US 2015/099726 A1, but with the electronics module 120 being omitted.

Regarding the mechanical structure of the inhaler and/or the deagglomerator, the disclosure of US 2015/099726 A1 is incorporated by reference in its entirety.

To the extent that any meaning or definition of a term in this document conflicts with any meaning or definition of the same term in a document incorporated by reference, such as US 2015/099726 A1, the meaning or definition assigned to that term in this document shall govern.

The present invention will now be described with reference to the following examples which are not intended to be limiting.

### Example 1 - fixed dose fluticasone propionate and albuterol sulfate formulations

### 55 mcg FP /117mcg ABS

0.1760 kg Fluticasone propionate (Fp) was blended together with 7.8240 kg of α-lactose monohydrate carrier for 10 min at 433 rpm (revolutions per minute) using a PharmaConnect high shear blender providing an 8 kg blend. 0.9400 kg of albuterol sulfate (ABS) was blended together with 19.0600 kg of α-lactose monohydrate carrier for 7 min at 300 rpm using a PharmaConnect high shear blender providing 20 kg of a blend. 8 kg of the Fp-containing blend and 8 kg of the ABS-containing blend were then mixed together using PharmaConnect low shear blender and mixed at 15 rpm for 20 mins to provide a final combination blend containing 1.1% w/w of fluticasone propionate (suitable for providing a 51 µg dose, size 3 dose cup) and 2.35% w/w of albuterol sulfate (suitable for providing a 90 µg albuterol base per dose, size 3 dose cup). The final combination blend was then filled into the reservoir of a dry powder inhaler device. The devices were subjected to 30°C/65%RH for 4 weeks for conditioning and then packed in a foil overwrap with a desiccant.

### 30 mcg FP /117mcg ABS

0.0873 kg of fluticasone propionate (Fp) was blended together with 7.9127 kg of α-lactose monohydrate carrier for 12 min at 150 rpm using a PharmaConnect high shear Blender providing 8 kg of a blend. 0.9400 kg of albuterol sulfate (ABS) was blended together with 19.0600 kg of α-lactose monohydrate carrier for 7 min at 300 rpm using a PharmaConnect high shear blender providing 20 kg of a blend. 8 kg of the Fp-containing blend and 8 kg of the ABS-containing blend were then mixed together using PharmaConnect low shear blender and mixed at 15 rpm for 20 min to provide a final combination blend containing 0.55% w/w of fluticasone propionate (suitable for providing a 26 µg dose, size 3 dose cup) and 2.35% w/w of albuterol sulfate (suitable for providing a 90 µg albuterol base per dose, size 3 dose cup). The final combination blend was then filled into the reservoir of a dry powder inhaler device. The devices were subjected to 30°C/65%RH for 4 weeks for conditioning and then packed in a foil overwrap with a desiccant.

### Example 2 - analytical methods

### Albuterol sulfate PSD measurement

### Test Method for Particle Size (Sympatec)

### Operating Conditions for the RODOS M Dry Powder Disperser and Aspiros

### Dispersion Method:

Air Pressure: 3.0 Bar
Feeder: Aspiros
Feed velocity/ rate: 18 mm/s
Lens: R3
Channel: N/A

### Trigger Condition

Start: c. optical > 2%
Valid: Always
Stop: 2s c. optical ≤ 2% or 5 seconds real time
Time Base: 2.0 ms

Approximately 50 mg of the dry powder ABS was weighed into Aspiros test tube following by inserting the tube into the Aspiros and running the method. This was then blown by compressed air via the RODOS M dry powder dispenser through the measuring zone triggering a measurement.

### Fluticasone propionate PSD measurement

### Measuring Parameters:

### Reagents:

De-ionized water Milli-Q
Tween 80 Reagent Grade
TA-10X FG defoamer N/A

### Equipment:

Instrument: Malvern Laser Diffraction Mastersizer 2000
Accessory name: Hydro 2000s

### Equipment Parameters:

Measuring Range: 0.02-2000 mcm
Model Type: General Purpose
Sensitivity: Normal
Particle Shape: Irregular
Mode: Wet Dispersion
Dispersant name: De-ionized water
Dispersant RI: 1.33
Material RI: 1.53
Absorption: 3.0
Measuring time: 10 seconds (10,000 snaps).
Background measuring time: 10 seconds (10,000 snaps).
Speed/stir speed: 2,000 rpm.
Aliquots: 1
Measurements: 3 per Aliquot.
Delay: 0 seconds between measurements.
Ultrasonic level: 100%
Obscuration: 10-30%
Measuring Procedure

### Sample Preparation for Wet Measurement:

Preparation of 1% (W/V) Tween 80: 30mL of de-ionized water were added into a clean 50 mL glass bottle containing a magnetic stirring bar. 0.3 g of Tween 80 was added to the glass vessel and then stirred for about 10 minutes until a homogeneous solution was obtained.

Preparation of 1% (W/V) TA-1 0X FG defoamer: 10 mL of de-ionized water was added into a clean 25 mL glass vial. Add 0.1 g of TA-10X FG defoamer. The vial was tightly closed with cap, then hand-shaken for about 30 seconds. The obtained emulsion was shaken for a couple seconds immediately before each usage.

Preparation for sampling: Approximately 1-10 g of sample was transferred into a glass bottle large enough so that the powder was freely tumbled to assure a homogenous sample throughout the bottle. The capped bottle was gently inverted up and down 10 times, and then rotated 10 times in a clockwise manner.

Preparation of the sample: About 50 mg of the sample powder was transferred with a spatula into a clean 25 mL glass vessel containing a magnetic stirring bar. 10 mL of de-ionized water containing 1% Tween 80 were added into the glass vessel. The suspension was stirred with the magnetic stirrer for 2 minutes at a moderate speed. The sample was then ready to be loaded into the wet dispersion unit.

### Manual measurement

The Hydro 2000S dispersion unit tank was manually filled with about 150 mL of de-ionized water. The ultrasonics was set at the level of 100% and sonicated for 30 seconds to allow air to dissipate, and then turned off. The pump/stirrer speed was turned up to full (3500 rpm), then down to zero to clear any bubbles.

About 0.3 mL of 1% TA-10X FG defoamer was added to the tank and then the pump/stirrer was resumed at 2000rpm. The background was measured by slowly dropping 1 out of 3 prepared samples from into the dispersion unit until a stabilized initial obscuration at 10-20% was reached. The sample was stirred in the dispersion unit for about 1 minute at 2,000 rpm and then turned on the ultrasound and the level was set to 100%. After about one minute the sample was measured and the result calculated.

### Lactose monohydrate PSD measurement

Instrument: Sympatec HELOS/BR Particle size Analyzer. Reference SOPM000564
Ensure testing is performed using the PSD composite sample
Set up the following operating conditions using the Vibratory Feeder and Funnel Adaptor
Instrument Settings
Parameter Setting
Sympatec Dispersion method: 1.5bar Vibri 75%
Lens type: R4
Dispenser: RODOS M
Measuring Range: R4: 0.5/1.8...350 mcm
Trigger Conditions
Name: Ch. 9 optical conc. ≥ 0.5%
Reference duration: 4s (single)
Time base: 100 ms
Focus prior to first measurement: Yes
Normal measurement: Standard mode*
Start: 0.000s, channel. 9 optical conc. ≥ 0.5%
Valid: Always
Stop after1 sec, channel. 9 optical conc. < 0.5%
Or after: 60.000s, real time
Repeat measurement: 0 times
Repeat focus: No
Disperser Conditions
Name: 1.5 bar; 75%; 2 mm
Dispersing type: RODOS/M
Injector: 4mm
With: 0 cascade elements
Primary pressure: 1.5 bar
Always auto adjust before reference measurement: No
Feeder type: VIBRI
Feed rate: 75%
Gap width: 2 mm
Funnel rotation: 0%
Cleaning time: 10s
Use VIBRI control: No
Vacuum extraction type: Nilfisk
Delay: 5s

The sample was mixed for 20 seconds by turning / rotating the sample. A suitable transfer container was used to weigh and transfer approximately 5.0 g of the sample from the composite container into the VIBRI funnel of the sample unit. This was then blown by compressed air via the RODOS dry powder dispenser through the measuring zone triggering a measurement.

### Example 3 - clinical trial

This example sets out a randomized, double-blind, multicenter, active-controlled, parallel-group study to evaluate the efficacy and safety of fluticasone propionate/albuterol sulfate fixed-dose combination on severe clinical asthma exacerbations in patients with asthma.

### General study design

This is an event-driven, phase 3, multicenter, randomized, double-blind, active-controlled, parallel-group, fixed dosage study to evaluate the efficacy and safety of a new combination product, Fp/ABS eMDPI (metered dose dry powder inhaler with an integrated electronic module), as a rescue medication compared to ABS eMDPI. The Fp/ABS eMDPI is evaluated at 2 dose strengths, a HD (55/117 mcg) and a LD (30/117 mcg) to assess severe CAE reduction compared to ABS eMDPI (117 mcg) in adult and pediatric patients (≥4 years of age) with asthma (baseline ICS level: low, medium, or high; GINA 2022).

Patients are 4 years of age or older (or as allowed by local regulation), with a clinical diagnosis of asthma for at least 1 year consistent with GINA 2022 and, as applicable, a smoking history of ≤10 pack-years. Patients have a documented history of at least 1 severe CAE over the past 12 months, demonstrate a ≥12% reversibility (or documented historical reversibility), and have an ACQ-5 score ≥1.5 during the screening assessment (except patients aged 4-5 years), despite taking a stable dose of prescribed inhaled asthma controller medications ±another controller (oral or inhaled). Patients taking prohibited medications including prohibited monoclonal antibodies, must comply with the washout periods (biologic medications used to treat asthma, 5 half-lives; depemokimab, any exposure prohibited; monoclonal antibodies used to treat malignancy, any exposure prohibited; any other investigational drug, 5 half-lives; corticosteroids (oral, iv, intra-articular, intramuscular), 30 days; immunologically active biologic medications, 90 days; immunosuppressive therapy, 90 days; immunotherapy, initiation within 90 days or change in dose within 30 days; strong cytochrome P3A4 inhibitors, 14 days; inhaled cromolyn preparations, 14 days; inhaled short-acting muscarinic antagonists (SAMA), discontinue at screening visit (v1); phosphodiesterase 4 inhibitors, 5 half-lives; monoamine oxidase inhibitors, 14 days; tricyclic anti-depressants, 14 days; and non-selective β₂-adrenergic blockers including eye medications, 14 days).

Patients continue to use their current prescribed asthma controller medication (not provided by the sponsor) throughout the study and are not permitted to change their controller medication if their asthma is stable, otherwise the change is considered a protocol deviation unless this change is due to formulary changes and the medication is similar (or an equivalent generic substitution) based on ICS dose (low, medium, or high) and after consultation with the medical monitor to gain agreement. Patients are randomized in a 1:1:1 ratio to receive either HD Fp/ABS eMDPI, LD Fp/ABS eMDPI, or ABS eMDPI to be used as rescue medication. Patients discontinue all other rescue therapies including nebulized SABA or short-acting muscarinic antagonists (SAMA). The IMPs are administered by blinded eMDPI oral inhalation (2 inhalations PRN to control asthma symptoms).

The study consists of a screening period of approximately 2 weeks, a run-in period (approximately 2 to 4 weeks), and a double-blind treatment period. Patients who experience a severe CAE remain on the study and continue to attend their scheduled visits.

As the study is event-driven, the end of study (EOS) is dependent on the number of severe CAEs first experienced by patients in both the HD Fp/ABS eMDPI and the ABS eMDPI groups. The study continues until 599 patients (at a minimum) experience a severe CAE in the two groups combined.

Once the study has accrued 599 first severe CAEs for HD Fp/ABS eMDPI and active control groups combined, patients who have completed visit 5 (V5) (24 weeks) are scheduled for an EOS visit within 30 days and patients who have been randomized but who have not completed V5 proceed in usual fashion until they complete V5 which serves as their EOS visit. The total study duration is estimated through simulations to be approximately 35 months in order to complete the study; however, due to the event-driven nature of this study, the duration may vary.

At the screening visit (visit 1 [V1]), informed consent (or assent for children 4 through <18 years of age or as required by local regulation) is obtained before other procedures are completed. The screening visit occurs on multiple days as required over approximately 2 weeks.

Pulmonary function testing (spirometry and reversibility testing) is conducted during screening in the morning between 0530 and 1100 hours using the on-site equipment (provided by the sponsor). Patients are required to withhold asthma maintenance medications for approximately 24 hours prior to lung function testing for once daily medications or 12 hours for twice daily, or more frequently dosed, medication. If the patient has taken asthma maintenance medication within 24±2 (daily dosed) or 12±2 hours (dosed twice daily or more frequently) or SABA (run-in rescue medication)/IMP within 6 hours of the planned pulmonary function testing or ICS/long-acting beta agonist (LABA) used as rescue medication within 12 hours of the planned pulmonary function testing, the visit is rescheduled.

Airway reversibility (response to bronchodilator testing), as measured by FEV1, at screening is demonstrated after pre-albuterol pulmonary function testing is completed via central spirometry and then repeating FEV1 measurement after receiving SABA medication. If a patient's FEV1 improves at least 12% between the 2 tests and an increase of 200 mL for patients ≥18 years of age (or has a documented history of ≥12% reversibility within the previous 18 months), that patient is assessed as having airway reversibility and continues the screening process. Note: one re-test is allowed during the screening period if needed to demonstrate the required pre and post albuterol study requirements. For patients ≥12 years of age, 4 inhalations of SABA medication are permitted for reversibility testing. Patients aged 4 to <12 years should use 2 inhalations of SABA or a single nebulized dose of albuterol (salbutamol) based on the investigator's judgement. All patients should attempt to demonstrate ≥12% reversibility even if historical reversibility is available, however patients less than 12 years of age may be included even if they do not demonstrate 12% response.

Patient medical history, asthma history including asthma medications and exacerbation history, physical examination, serum chemistry, urine drug screen, vital sign measurements, beta-human chorionic gonadotropin (β-HCG) serum pregnancy test (for all females of childbearing potential [CBP]), serum follicle stimulation hormone (FSH) for postmenopausal women only, complete blood count (CBC) with differential, and concomitant medication history is also assessed at screening. Note: documentation of asthma exacerbation within the previous 12 months must be obtained. This includes medical or hospital records or patient provided evidence of prescriptions for SCS used during an exacerbation. Patients who otherwise qualify may enter the run-in period while attempting to collect medical records for documentation of asthma exacerbations but are not randomized at V3 if the records have not been obtained and verified.

Patients meeting all the eligibility criteria continue with their current prescribed asthma controller medication, but discontinue current rescue medication. Patients are given study-specific rescue medication (ProAir^{®} RespiClick^{®} or equivalent) and subsequently enrolled in the run-in period (visit 2 [V2]) of the study. Patients are provided and trained on the use of an electronic diary (eDiary), trained on an empty inhaler device, and trained on a handheld device to measure lung function. During the run-in period, patients measure FEV1 (forced expiratory volume measured during the first second following maximal inhalation) and PEF (peak expiratory flow) each morning using the handheld device prior to the use of run-in rescue medication (whenever possible) and prior to the taking their usual asthma maintenance medication(s). Patients also record asthma (daytime or night-time) symptom score and rescue (i.e. IMP once randomized) medication (number of inhalations) twice daily whether used or not, and confirm each evening if they took their routine asthma maintenance medication (yes or no) that day. Patients without a disqualifying event during the run-in period will receive the remaining baseline evaluations.

Patients may be evaluated at V3 for randomization after a minimum of 14 days and up to 31 days (28±3 days) of participation in the run-in period (V2 to V3). In addition to confirmation of inclusion/exclusion criteria, baseline procedures and assessments are to be performed prior to randomization in the randomization and trial supply management (RTSM) system, before IMP dispensing and will include a full physical examination; vital sign measurements; review of diary data to confirm eligibility; establishment of baseline pulmonary function testing; training for correct inhaler use with the empty demo eMDPI inhaler device or as specified in the pharmacy manual (training inhaler), and evaluation of use; perform ACQ-5 assessment and confirm ACQ-5 score ≥1.5 (patients aged 4-5 will not complete the ACQ-5); urine pregnancy test (for all female patients of CBP); ECG recording; concomitant medication inquiry (including birth control review); CAE inquiry; and adverse event inquiry.

Patients who are randomized into the study are randomly assigned in a blinded fashion to 1 of the 3 treatment groups (HD Fp/ABS eMDPI, LD Fp/ABS eMDPI 3, or ABS eMDPI) at the baseline visit. Patients are stratified by geographical region, age group, and number of exacerbations in the prior year at screening (indicative of ongoing controller medications) across all 3 treatment groups to ensure similar distribution of patients. Randomly assigned patients receive IMP to control asthma symptoms, 2 inhalations PRN to relieve asthma symptoms, throughout the treatment period and record daily use in an eDiary, whether used or not, along with daily morning FEV1 and PEF measured at home. Patients return to the investigational center per a pre-specified visit (quarterly [i.e. every 12 weeks]) schedule, and phone assessments are conducted approximately monthly between investigational center visits. Patients continue in the study with quarterly visits and monthly phone contacts until the number of exacerbations have occurred. Randomized patients who have not completed at least 24 weeks of the treatment period should continue until they complete V5 even if the required number of exacerbations have occurred. Pulmonary function testing, AQLQ+12 (or PAQLQ), ACQ-5 (ages ≥6 years only), eDiary review (asthma symptoms and compliance), and safety monitoring (adverse events, vital sign measurements, physical examinations, and concomitant medication usage) are performed throughout the study. In addition to safety monitoring by the sponsor, an IDMC will regularly review safety data.

Alert criteria for worsening asthma have been developed to ensure patient safety and are monitored throughout the study from V2 onward. If any of the criteria listed below are met or there has been no data transmitted for 3 consecutive days, the investigator, the clinical research center personnel, and medical monitor at a minimum are notified automatically via the portal. Meeting any of these criteria does not automatically require a patient to be treated with additional therapy, rather it requires a clinical evaluation to determine if the patient's asthma can continue to be managed on the current regimen, requires a change in maintenance medication or represents a severe CAE requiring treatment.

The alert is generated if any 1 or more of the following occur on at least 2 consecutive days based on the daily diary data:
- Morning PEF falls below the PEF stability limit. The PEF <80% of the baseline value determined at V3 (average of the highest daily morning PEF [from 3 attempts each day] over the 7 days prior to V3).
- Nighttime asthma symptom score that is greater than the baseline score and ≥2. The baseline score is the average of the score over the 7 days prior to V3 rounded to the nearest whole number.
- Daytime asthma symptom score that is greater than baseline and ≥3. The baseline score is the average of the score over the 7 days prior to V3 rounded to the nearest whole number.
- Rescue medication (i.e. IMP) use >4 inhalations per day and ≥2 inhalations greater than baseline (baseline is defined as the average number of inhalations/day over the 7 days prior to V3 rounded to the nearest whole number).

Patients who meet these criteria and require a change in asthma medication or experience a severe CAE are considered to have a deterioration of asthma and this is considered one event until the criteria are no longer met or until they become unevaluable. Each event is counted once until at least 3 days occur without meeting criteria. Additionally, when patients who, by the assessment of the investigator, are identified as having experienced a clinically meaningful deterioration of asthma, even if they do not meet the criteria above, the investigator must document the clinical reason for this designation.

During the treatment period, patients are contacted approximately monthly (every 4 weeks) by phone to assess CAEs, adverse events, concomitant medications, and to confirm diary compliance after reviewing compliance to diary data in the portal.

### Primary and Secondary Study Objectives and Endpoints

The primary and secondary study objectives and endpoints are:

| *Objectives* | *Endpoints* |
|---|---|
| The primary objective of the study is to demonstrate the efficacy of high dose (HD) and low dose (LD) fluticasone propionate (Fp)/albuterol sulfate (ABS) integrated electronic module multidose dry powder inhaler (eMDPI) compared to ABS eMDPI in decreasing severe CAEs when used as a rescue medication. | The primary efficacy endpoint is the time to first severe CAE (a worsening [deterioration] of asthma) |

| *Objectives* | *Endpoints* |
|---|---|
| A secondary objective of the study is to evaluate the efficacy of Fp/ABS eMDPI 2 inhalations taken as needed (PRN) compared to that with ABS eMDPI 2 inhalations PRN and the effect on systemic corticosteroid (SCS) exposure. | The secondary efficacy endpoints are: |
| | - annualized severe CAE rate |
| | - total annualized SCS exposure over the treatment period |
| | - Asthma Control Questionnaire-5 (ACQ-5) response at week 24, defined as achieving a decrease in score from baseline value of at least 0.5 (patients aged ≥6 years) |
| | - AQLQ+12/PAQLQ response at week 24, defined as achieving an increase in score from baseline of at least 0.5 (patients aged ≥7 years) |
| A secondary objective of the study is to evaluate the safety and tolerability of Fp/ABS eMDPI 2 inhalations PRN over 24 weeks. | The safety/tolerability endpoints are: |
| | - occurrence of adverse events during the study |
| | - occurrence of serious adverse events during the study |
| | - study withdrawal due to treatment-emergent adverse events |

The time to first severe CAE event is a clear clinical measure of benefit relevant to asthmatics at all levels of severity. From a clinical trial perspective, it obviates the potential for individual patients to contribute multiple events to the primary analysis encountered with time weighted event numbers. Its use is consistent with the American Thoracic Society's 2009 statement on asthma control and exacerbations with consensus recommendations on standardized definitions for assessment of asthma exacerbations in clinical trials.

The primary estimated for the study is defined by the attributes below:

| | |
|---|---|
| Treatment | Patients are randomly assigned to receive one of the study treatment regimens shown below in a 1:1:1 ratio: |
| | • HD Fp/ABS eMDPI 55/117 mcg |
| | • LD Fp/ABS eMDPI 30/117 mcg |
| | • ABS eMDPI 117 mcg (active control) |
| Target population | Eligible male and female patients ≥4 years of age diagnosed with asthma for at least 1 year |
| Primary outcome measure | The primary efficacy measure for the study is the time to first severe CAE. Time to first severe CAE is calculated as the time from randomization until the start date of the first severe CAE. An |
| | asthma exacerbation is considered severe if it results in at least 1 of the following: |
| | - treatment with SCS for at least 3 consecutive days to treat symptoms of asthma worsening. A single depo-injection of corticosteroids are considered equivalent to a 3-day course of oral corticosteroids |
| | - an urgent care or emergency room visit due to asthma that required SCS for at least 3 consecutive days |
| | - a hospitalization (defined as admission to an in-patient facility or observation unit) for ≥24 hours due to asthma |
| Intercurrent events strategy | The following are considered as intercurrent events (ICEs): |
| | - occurrence of COVID-19 pneumonia that is clinically diagnosed and requiring systemic therapy or with chronic respiratory sequelae that might change the natural history of the patient's airway disease |
| | - any use of SCS or other biological therapy for other diseases |
| | - any change in patients' asthma controller therapy |
| | These events do not preclude follow-up for severe CAE (i.e. the treatment policy strategy is adopted). |
| Population-level summary | The population-level summary of interest is the hazard ratio comparing the time to first severe CAE for HD Fp/ABS eMDPI vs active control and for LD Fp/ABS eMDPI vs active control |

| | |
|---|---|
| ABS=albuterol sulfate; CAEs=clinical asthma exacerbations; COVID-19=Coronavirus Disease 2019; eMDPI=electronic module multidose dry powder inhaler; Fp=fluticasone propionate; GINA=Global Initiative for Asthma; HD=high dose; ICE=intercurrent events; LD=low dose; SCS=systemic corticosteroid | |

### Exploratory endpoints

The exploratory objective of the study is to characterize the pattern of rescue treatment (i.e. IMP) utilization of HD or LD Fp/ABS eMDPI compared to ABS eMDPI and it will be assessed with the following endpoints:
- vital signs, physical examination (including oropharyngeal), clinical laboratory tests, and ECG findings
- concomitant medication use throughout the study
- Pattern of study medication use (daily number of inhalations is determined based on patient's eDiary)
- ACQ-5 change from baseline (visit 3 [V3]) at week 12 (patients aged ≥6 years)
- ACQ-5 response at week 12, defined as achieving a decrease from baseline of at least 0.5 (Patients aged ≥6 years)
- AQLQ+12/PAQLQ change from baseline at week 12 (PAQLQ for patient 7 to <12 years of age)
- AQLQ+12/PAQLQ change from baseline at week 24 (PAQLQ for patient 7 to <12 years of age)
- AQLQ+12/PAQLQ response at week 12, defined as achieving an increase from baseline of at least 0.5 (PAQLQ for patient 7 to <12 years of age)
- trough FEV1 change from baseline at week 12 (patients aged ≥12 years of age)
- trough percent predicted FEV1 change from baseline at week 12 (patients aged ≥4 years)
- trough FEV1 change from baseline at week 24 (patient aged ≥12 years of age)
- change from baseline in the weekly average of the daily morning FEV1 measured by the handheld device over weeks 1 to 24 (patients ≥12 years). Note: baseline here is the average daily morning FEV1 over the 7 days prior to V3.
- trough percent predicted FEV1 change from baseline at week 24 (patient aged ≥4 years). Baseline is the average of the percent predicted FEV1 over the 7 days prior to V3.
- change from baseline in the weekly average of the daily morning peak expiratory flow (PEF) measured by the handheld device over weeks 1 to 24. Note: baseline here is the average of the morning PEF over the 7 days prior to V3.
- change from baseline in the weekly average of the total daily asthma symptom score (defined as the average of the daytime and nighttime scores) over weeks 1 to 24. Note: baseline here is the average score over 7 days prior to V3.
- percentage of symptom-free days (defined as 24-hour periods with asthma symptom score of 0) over 24 weeks
- percentage of Asthma Control Days (defined as 24-hour period with asthma symptom scores of zero and no rescue [i.e. IMP] medication use over 24 weeks)
- percentage of rescue (i.e. IMP) medication free days (defined as days where no rescue [i.e. IMP] was used over 24 weeks)
- time to first deterioration in asthma requiring a change in asthma treatment (includes patients who experience a severe CAE)
- change from baseline in the weekly average of daily (24 hour) use of rescue medication (i.e. IMP) (number of inhalations) at week 24. Note: baseline here is the average number of inhalations over the 7 days prior to V3.

### Planned number of patients and countries

Approximately 4880 patients are screened to achieve approximately 2196 randomized patients (-10% pediatric patients aged 4 to <12 years and -10% adolescent patients aged 12 to <18).

The total number of patients planned is approximately 732 patients per treatment group.

### Patient inclusion criteria

Patients are included in this study only if they meet all of the following criteria:
(a) The patient is capable of giving signed informed consent (age ≥18 years). Patients ages 4 to <18 years (or as applicable and required by local regulation) are able to provide assent and written consent must be provided by a parent/legally acceptable representative before any study procedures are performed.
(b) The patient is a male or female 4 years of age or older at the time of informed consent/assent or age as allowed by local regulation.
(c) The patient has a diagnosis of asthma for at least 1 year according to the 2022 GINA guidelines.
(d) The patient has an ACQ-5 score of ≥1.5 (patients aged 4-5 years are not required to meet this criteria).
(e) The patient has a documented history of at least 1 severe CAE within the past 12 months before screening.
   Note: Documentation of asthma exacerbation within the previous 12 months must be obtained. This includes medical or hospital records or patient provided evidence of prescriptions for SCS used during an exacerbation. Patients who otherwise qualify may enter the run-in period while attempting to collect medical records for documentation of asthma exacerbations but must not be randomized at visit 3 (V3) if the records have not been obtained and verified
(f) The patient is using any prescribed inhaled asthma controller medication (at a stable dose for 1 month prior to the screening visit). Examples include the following:
   - low- to high-dose ICS
   - low- to high-dose ICS and 1 additional maintenance therapy from the following: leukotriene receptor antagonists (LTRA), long-acting muscarinic antagonists (LAMA), or theophylline
   - low- to high-dose ICS in combination with LABA with or without 1 additional maintenance therapy from the following: LTRA, LAMA, or theophylline
   - patients aged 4 to <6 years may participate if they meet the minimum ICS dosage as specified in the "allowed medication" section.
(g) The patient has a prebronchodilator FEV1 of ≥40 to <90% predicted normal value for patients ≥12 years of age, and ≥60 to <100% predicted normal value (Quanjer et al. 2012) for patients 4 through 11 years of age after withholding specified medications including SABA (run-in rescue medication) for spirometry testing.
(h) The patient demonstrates proper technique using the training inhaler during the run-in period (V2) and prior to IMP dispensing (V3), as per the instructions for use. Note: Spacer devices are prohibited for use with the eMDPI device to be used on study, as they are incompatible.
(i) The patient demonstrates age-appropriate acceptable spirometry performance during screening (i.e. meets American Thoracic Society/European Respiratory Society acceptability/repeatability criteria) (Graham BL et al. 2019). Note: Two acceptable and repeatable curves are adequate for patients aged 4 to <12 years of age.
(j) The patient is capable of performing PEF/FEV1 measurements using the handheld device as judged by the investigator.
(k) Bronchodilator responsiveness testing ("reversibility testing"): the patient (for patients 12 years of age and older) demonstrates airway reversibility following a beta-2 agonist challenge with an increase in FEV1 (≥12%) relative to baseline via central spirometry after administration of sponsor provided albuterol. Patients aged 18 and older must also demonstrate a 200 ml improvement from baseline FEV1. One retest for reversibility is allowed during the screening period in advance of the second visit. Or

The patient has a documented history of ≥12% airway reversibility of FEV1 within the previous 18 months.

Note: All patients including patient ages 4 to 11 years must attempt reversibility testing.
(I) The patient must be willing and able to comply with study restrictions and to remain at the investigational center for the required duration during the study period and any follow-up procedures and assessments as specified, and be willing to return to the investigational center for further visits, as applicable.
(m) If female, the patient is currently not pregnant, breastfeeding, or attempting to become pregnant (for at least 30 days before the screening visit and throughout the duration of the study), or is of non CBP, defined as any of the following:
   - premenarche
   - at least 1 year postmenopausal (no menses for at least 12 months without an alternative medical cause plus an increased concentration of FSH of more than 35 U/L in women not using hormonal contraception or hormonal replacement therapy)
   - surgically sterile (bilateral tubal ligation, bilateral oophorectomy, salpingectomy, or hysterectomy)
   - congenitally sterile (as assessed by a physician)
   - diagnosed as infertile and not undergoing treatment to reverse infertility

Or, if of CBP, has a negative serum β-HCG test at screening visit and is willing to commit to using a medically accepted, highly effective method of birth control as defined below for the duration of the study:
- combined estrogen and progestogen hormonal contraception (oral, intravaginal, transdermal) associated with inhibition of ovulation; these should be initiated at least 30 days before the first dose of IMP and maintained throughout the study.
- progestogen-only hormonal contraception (oral, injectable, implantable) associated with inhibition of ovulation; these should be initiated at least 30 days before the first dose of IMP and maintained throughout the study.
- intrauterine device and intrauterine hormone-releasing system need to be in place at least 2 months before screening and maintained throughout the study.
- bilateral tubal occlusion, except for hysteroscopic bi-tubal ligation for which a hysterosalpingogram is required 3 months post procedure to assess surgical success
- vasectomized partner provided he is the sole sexual partner and has received medical assessment of the surgical success
- sexual abstinence - refraining from heterosexual intercourse during the study period

Of note, if female patient has reached puberty and achieved menarche (as determined by the investigator), the patient must be counseled regarding the possible unknown risks associated with IMP during pregnancy (following permission of the parent/legally acceptable representative as required).

### Investigational medicinal products used in the study

The IMPs used in this study are defined as the test IMP and the active control. The active control is authorized and is used in accordance with the terms of the marketing authorizations in respective countries (although ProAir^{®} is only presently approved in the US and Canada, and ProAir^{®} Digihaler^{®} is only presently approved in the US).

### Test investigational medicinal product

The test IMP is Fp/ABS eMDPI inhalation powder, which is further described in the table hereinbelow.

Patients receive the test IMP at a HD (55/117 mcg) or LD (30/117 mcg) with a dosing frequency of 2 inhalations PRN to control asthma symptoms. The maximum daily dose in the trial is 12 inhalations (6 doses) per day. The number of occasions when patients exceed 12 inhalations (6 doses) in a day are reported in the study report.

Patients are also advised to rinse their mouth with water without swallowing, after inhalation.

### Active control

The active control is ABS eMDPI inhalation powder, which is further described in the table hereinbelow. Additional details may be found in the PI (patient information) for ProAir^{®} Digihaler^{®}, June 2022 and ProAir^{®}RespiClick^{®}, September 2020.

Patients will receive the active control at a dosing frequency of 2 inhalations PRN (delivered dose is 108 mcg each) to control asthma symptoms.

The maximum daily dose is 12 inhalations (6 doses) per day. The number of occasions when patients exceed 12 inhalations (6 doses) in a day are reported in the study report.

Patients are also advised to rinse their mouth with water without swallowing, after inhalation.

### Auxiliary medicinal product

During the run-in period, patients are provided ProAir^{®} RespiClick^{®} (albuterol [salbutamol]) inhalation powder 117 mcg/inhalation) for use as rescue medication (as allowed by local regulation) and is considered auxiliary medicinal product. The formulation is the same as the active control for this study. The use of this platform during the run-in period (2-4 weeks) will allow the patients to become familiar with proper use of the device after training. The same device platform is used during the treatment period of the study where the IMPs are used as rescue medication. The use of ProAir^{®} RespiClick^{®} during the run-in period also allows for standardization of treatment across the several geographies providing for a consistent study design.

### Inhaler device

The inhaler device manufactured by Teva provided for use in this study is the eMDPI (Digihaler) device, as described herein, which contains a built-in eModule that detects, records, and stores data on inhaler events.

In the current study, the eModule component will not be used in conjunction with a mobile application. The inhalers are returned to a central site and the data are downloaded in compliance with Global Data Privacy.

All patients are provided training on use of a training inhaler device during V2 and V3. Note: patients must be able to use the device without a spacer device due to incompatibility. Spacers are not allowed for use with the eMDPI device. The training is done after the screening visit when the patient has been deemed eligible for inclusion and is commencing the run-in period. Directions for use of the device are reviewed with each patient. Patients are then provided with an inhaler training device. The training devices are empty and do not contain active medication. Patients practice using the device and are required to demonstrate their understanding of the correct technique. To ensure the correct technique for the device, it is used over the duration of the study, training procedures are repeated and documented at each clinic visit for patients who demonstrate incorrect technique during the evaluation of inhaler use. Sites are responsible for labeling and storing the training inhaler devices between visits (further detailed in the study reference manual).

Instructions on proper usage of the inhaler(s) are provided by the sponsor.

Investigational medicinal products and device used in the study

| | Test IMP | Active Control |
|---|---|---|
| IMP Name | Fp/ABS eMDPI | ABS eMDPI ProAir^{®} Digihaler^{®} (study blinded version) |
| Formulation | Inhalation powder | Inhalation powder |
| Unit dose strength(s)/Dosage level(s) | 30/117 mcg: | 117 mcg: |
| | 30 mcg of Fp from the device reservoir to produce a delivered dose of 26 mcg Fp per actuation and 117 mcg of ABS per inhalation from the device reservoir to provide a delivered dose of 108 mcg of ABS (equivalent to 90 mcg of albuterol base) per actuation | 117 mcg of ABS per inhalation (equivalent to 97 mcg of albuterol base) from the device reservoir to provide a delivered dose of 108 mcg of ABS (equivalent to 90 mcg of albuterol base) per actuation |
| | 55/117 mcg: | |
| | 55 mcg of Fp from the device reservoir to produce a delivered dose of 51 mcg Fp per actuation and 117 mcg of ABS per inhalation from the device reservoir to provide a delivered dose of 108 mcg of ABS (equivalent to 90 mcg of albuterol base) per actuation | |
| Route of Administration | Oral Inhalation | Oral Inhalation |
| Dosing instructions | 2 inhalations PRN | 2 inhalations PRN |
| Device type | Standard flow | Standard flow |
| Packaging | Inhaler device | Inhaler device |
| | Test IMP | Active Control |
| Storage | Room temperature (15-25°C; 59-77°F) | Room temperature (15-25°C; 59-77°F) |
| | | |

| | | |
|---|---|---|
| ABS=albuterol sulfate; eMDPI=multidose dry powder inhaler with integrated electronic module; Fp=fluticasone propionate; IMP=Investigational Medicinal Product. | | |

### Allowed medications

The following medications in the table are permitted, but with restrictions, during the study:

List of ICS doses allowed for inclusion into the study for patients ≥12 years of age

| Asthma maintenance therapy^{a} | Daily Dose (mcg/day) | Daily dose (mcg/day) | Daily dose (mcg/day) |
|---|---|---|---|
| | Low Dose | Medium dose | High dose |
| Fluticasone propionate HFA pMDI | 100-250 mcg (metered dose) | >250 mcg (metered dose^{b}) | >500 mcg (metered dose) |
| | 88-220 mcg (delivered dose | >220 mcg (delivered dose) | > 440 mcg (delivered dose) |
| Fluticasone propionate DPI | 200 mcg (metered dose) | >250 mcg (metered dose) | >500 mcg (metered dose) |
| Fluticasone propionate DPI (ArmonAir^{®}/AirDuo^{®})^{c} | 110 mcg (metered dose) | 226 mcg (metered dose) | 464 mcg (metered dose) |
| Fluticasone furoate DPI | - | 100 mcg | 200 mcg |
| Beclomethasone dipropionate DPI (EASYHALER^{®})^{d} | 200-400 mcg (metered dose) | >500 mcg (metered dose) | >1000 mcg (metered dose) |
| Beclomethasone dipropionate HFA pMDI (QVAR^{®})^{e} | 100-200 mcg (metered dose) | >200 mcg (metered dose) | >400 mcg (metered dose) |
| | 80-160 mcg (delivered dose) | >160 mcg (delivered dose) | >320 mcg (delivered dose) |
| Budesonide DPI | 400 mcg (metered dose) | >400 mcg (metered dose) | >800 mcg (metered dose) |
| | 360 mcg (delivered dose) | >360 mcg (delivered dose) | >720 mcg (delivered dose) |
| Mometasone furoate DPI^{f} | - | 200 mcg | 400 mcg |
| Mometasone furoate HFA pMDI | 200 mcg | >200 mcg | >400 mcg |
| Ciclesonide HFA pMDI | 80-160 mcg (delivered dose) | >160 mcg (delivered dose) | >320 mcg (delivered dose) |

| | | | |
|---|---|---|---|
| a Does not include ICS/LABA used as rescue medication b Consult prescribing information to determine metered or delivered dose if required. c ArmonAir^{®} Respiclick^{®} USPI (revised July 2021), ArmonAir^{®} Digihaler^{®} USPI (revised March 2022), and AirDuo RespiClick^{®} USPI (revised July 2021), AirDuo Digihaler^{®} USPI (revised July 2021) d EASYHALER beclometasone SmPC (revised April 2017). e QVAR REDIHALER USPI (revised January 2021), QVAR 100 Autohaler SmPC (revised November 2019), and QVAR 50 Easi-Breathe SmPC (revised November 2020). f Depends on DPI device - see product information. DPI=dry powder inhaler HFA=hydrofluoroalkane; ICS=inhaled corticosteroid; LABA=long-acting beta agonist; pMDI=pressurized metered dose inhaler; SmPC=Summary of Product Characteristics; USPI=United States Prescribing Information. Note: any dose including low dose ICS/LABA fixed-dose combinations are allowed. Inhaled long-acting muscarinic antagonist are allowed if part of patients' background medication at the time of screening. Source: GINA 2022. | | | |

List of ICS doses allowed for inclusion into the study for patients 6 <12 years of age

| Asthma maintenance therapy | Daily dose (mcg/day) | Daily dose (mcg/day) | Daily dose (mcg/day) |
|---|---|---|---|
| | Low dose | Medium dose | High dose |
| Beclomethasone dipropionate (CLENIL MODULITE^{®})^{a} | 100-200 mcg | >200-400 mcg (metered dose) | >400 mcg (metered dose) |
| Beclomethasone dipropionate DPI (EASYHALER^{®})^{b} | -- | >200-400 mcg (metered dose) | >400 mcg (metered dose) |
| Beclomethasone dipropionate HFA (QVAR^{®})^{c} | 100-200 mcg (metered dose) | > 100-200 mcg (metered dose) | >320 mcg (delivered dose) |
| | 80-160 mcg (delivered dose) | >80-160 mcg (delivered dose) | >400 mcg (metered dose) |
| Budesonide DPI | 100-200 mcg (delivered dose) | >200-400 mcg (metered dose) | >400 mcg (metered dose) |
| Ciclesonide HFA MDI | 80 mcg (delivered dose) | >80-160 mcg (delivered dose) | >160 mcg (delivered dose) |
| Fluticasone furoate DPI | -- | 50 mcg | n.a. |
| Fluticasone propionate DPI | 50-100 mcg (metered dose) | >100-200 mcg (metered dose) | >200 mcg (metered dose) |
| Fluticasone propionate DPI Armonair^{®} Respiclick^{®}, Armonair^{®} Digihaler^{®} | 60 mcg (metered dose) | | 110 mcg (metered dose) |
| Fluticasone propionate HFA MDI | 50-100 mcg (metered dose) | >100-200 mcg (metered dose) | >200 mcg (metered dose) |
| | 44-88 mcg (delivered dose) | | |
| Mometasone furoate HFA MDI | -- | 100 mcg | 200 mcg |
| Mometasone furoate DPI | -- | 110 mcg (metered dose) | -- |
| | | 100 mcg (delivered dose) | |
| Budesonide suspension for nebulization | 250-500 mcg | >500-1000 mcg | >1000 mcg |

| | | | |
|---|---|---|---|
| a CLENIL MODULITE SmPC (revised December 2018). b EASYHALER beclometasone SmPC (revised April 2017) c QVAR REDIHALER USPI (revised January 2021), QVAR 100 Autohaler SmPC (revised November 2019), and QVAR 50 Easi-Breathe SmPC (revised November 2020). DPI=dry powder inhaler; HFA=hydrofluoroalkane; ICS=inhaled corticosteroid; MDI=metered dose inhaler; SmPC=Summary of Product Characteristics; USPI=United States Prescribing Information. Source: GINA 2022. | | | |

List of ICS doses allowed for inclusion into the study for patients 4-5 years of age

| Inhaled Corticosteroid | Low total daily dose (mcg/day) |
|---|---|
| Beclomethasone dipropionate (MDI, standard particle, HFA) | 100 mcg ≥5 years of age) |
| Beclomethasone dipropionate HFA (extrafine particle) | 100 (metered dose) |
| | 80 (delivered dose) |
| Budesonide suspension for nebulization | 250 mcg |
| Fluticasone propionate HFA MDI | 100mcg (metered dose) |
| | 88mcg (delivered dose) |
| Fluticasone propionate DPI | 60 mcg (metered dose) |
| Armonair^{®} Respiclick^{®}, Armonair^{®} Digihaler^{®} | |
| Mometasone furoate MDI HFA (standard particle) | 100 mcg ≥5 years of age) |
| Mometasone furoate DPI | 110 mcg |
| Fluticasone furoate DPI | 50 mcg ≥5 years of age) |
| Ciclesonide HFA MDI | Safety not established |

| | |
|---|---|
| DPI=dry powder inhaler; HFA=hydrofluoroalkane; ICS=inhaled corticosteroid; MDI=metered dose inhaler. Source: GINA 2022. | |

In addition to the above, the following medications are also permitted, but with restrictions, during the study:
- Chronic and PRN doses of low potency topical corticosteroids (e.g. 1% hydrocortisone cream, desonide, fluocinolone cream 0.01%) are permitted for dermatologic diseases, not to exceed 20% of the body surface area. Topical corticosteroids of any strength with an occlusive dressing are prohibited.
- Chronic stable doses of intranasal corticosteroids are allowed at least 30 days' duration before V1. Initiation of intranasal corticosteroids during the study is prohibited.
- Chronic stable doses of ocular steroids of at least 30 days duration are allowed at least 30 days duration before the V1.
- Immunotherapy for the treatment of allergies by any route is permitted as long as therapy was initiated 90 days or more before the V1 and the patient has been on a stable dose for 30 days or more before the V1. The regimen must remain stable throughout the study.

## Claims

1. A method of treatment of asthma comprising a pro re nata (PRN) administration of a fixed-dose dry powder inhalation composition comprising fluticasone propionate and albuterol sulfate as a rescue medication in patients aged from ≥4 years to <18 years.

2. The method of claim 1, wherein the asthma treatment treats or prevents bronchospasm in patients aged ≥4 years to <18 years with reversible obstructive airways disease.

3. The method of claim 1, wherein the asthma treatment prevents exacerbations in patients aged ≥4 years to <18 years old.

4. The method of claim 1, wherein the asthma treatment comprises one or more of prolonging the time to first severe clinical asthma exacerbation (CAE) when compared to albuterol sulfate rescue treatment, reducing the total annualized systemic corticosteroid (SCS) exposure when compared to albuterol sulfate rescue treatment and reducing the annualized severe clinical asthma exacerbation (CAE) rate when compared to albuterol sulfate rescue treatment.

5. The method of claim 4, wherein the asthma treatment comprises one or more of prolonging the time to first severe clinical asthma exacerbation when compared to albuterol sulfate rescue treatment and reducing the total annualized systemic corticosteroid (SCS) exposure when compared to albuterol sulfate rescue treatment.

6. The method of claim 4, wherein the asthma treatment comprises one or more of reducing the total annualized systemic corticosteroid (SCS) exposure when compared to albuterol sulfate rescue treatment and reducing the annualized severe clinical asthma exacerbation (CAE) rate when compared to albuterol sulfate rescue treatment.

7. The method of claim 4, wherein the asthma treatment comprises one or more of prolonging the time to first severe clinical asthma exacerbation when compared to albuterol sulfate rescue treatment and reducing the annualized severe clinical asthma exacerbation (CAE) rate when compared to albuterol sulfate rescue treatment.

8. The method of claims 1 or 4, wherein the asthma treatment comprises one or more of prolonging the time between severe clinical asthma exacerbations when compared to albuterol sulfate rescue treatment and decreasing the amount of time a patient has a severe clinical asthma exacerbation when compared to albuterol sulfate rescue treatment.

9. The method of claims 1, 4 or 8, wherein the treatment of asthma provides at least one of the following: achieving a decrease in the score from baseline value of at least 0.5 in the Asthma Control Questionnaire-5 (ACQ-5) response at week 24 when compared to albuterol sulfate rescue treatment.; and achieving an increase in the score from baseline of at least 0.5 in the AQLQ+12/PAQLQ (patients aged ≥7 years) response at week 24 when compared to albuterol sulfate rescue treatment.

10. The method of claim 9, wherein the treatment of asthma is in patients aged 6 to <18 years old.

11. The method of claim 1, wherein the patients are separately administered a maintenance dose of a long-term asthma medication.

12. The method of claim 11, wherein the long-term asthma medication is an ICS, LABA, LAMA, SAMA, biological medication or combinations thereof.

13. The method of claim 12, wherein the ICS is selected from flunisolide, fluticasone propionate, fluticasone furoate, beclomethasone dipropionate, budesonide, mometasone furoate, ciclesonide and combinations thereof.

14. The method of claim 13, wherein the LABA is selected from formoterol, arformoterol, salmeterol, bambuterol, clenbuterol, abediterol, indacaterol, olodaterol, vilanterol, carmoterol and combinations thereof.

15. The method of claim 13, wherein the LAMA is selected from tiotropium, glycopyrronium, umeclidinium, aclidinium, darotropium and combinations thereof.

16. The method of claim 13, wherein the SAMA is ipratropium.

17. The method of claim 11 wherein the maintenance therapy daily metered dose is low-, medium- or high-dose depending on the type of medicament and patient's age.

18. The method of claim 17, wherein for patients aged 12 years <18 years old the ICS daily metered doses are: beclomethasone dipropionate, low-dose 200-500 mcg, medium-dose >500-1,000 mcg, high-dose >1,000 mcg; beclomethasone dipropionate ultra-fine particle, low-dose 100-200 mcg, medium-dose >200-400 mcg, high-dose >400 mcg; budesonide, low-dose 200-400 mcg, medium-dose >400-800 mcg, high-dose >800 mcg; ciclesonide, low-dose 80-160 mcg, medium-dose >160-320 mcg, high-dose >320 mcg; fluticasone furoate, low-dose and medium-dose 100 mcg, high-dose 200 mcg; fluticasone propionate, low-dose 100-250 mcg, medium-dose >250-500 mcg, high-dose >500 mcg; and mometasone furoate, low-dose and medium-dose 200-400 mcg, high-dose is >400 mcg.

19. The method of claim 17, wherein for patients aged 6 to <12 years old the ICS daily metered doses are: beclomethasone dipropionate, low-dose 100-200 mcg, medium-dose >200-400 mcg, high-dose >400 mcg; beclomethasone dipropionate ultra-fine particle, low-dose 50-100 mcg, medium-dose >100-200 mcg, high-dose >200 mcg; budesonide, low-dose 100-200 mcg, medium-dose >200-400 mcg, high-dose is >400 mcg; budesonide nebules, low-dose 250-500 mcg, medium-dose >500-1,000 mcg, high-dose >1,000 mcg; ciclesonide, low-dose 80 mcg, medium-dose >80-160 mcg, high-dose >160 mcg; fluticasone furoate, low-dose and medium-dose 50 mcg; fluticasone propionate, low-dose 50-100 mcg, medium-dose >100-200 mcg, high-dose >200 mcg; and mometasone furoate, low-dose and medium-dose 100 mcg, high-dose 200 mcg

20. The method of claim 1, wherein patients aged from ≥4 years to <18 includes at least one of ≥4 to <12-year-old patients, ≥6 to <12year old patients ≥12 to <18year old patients and combinations thereof.

21. The method of claim 20, wherein the patients are ≥4 to <12 years old.

22. The method of claim 20, wherein the patients are ≥6 to <12 years old.

23. The method of claim 20 wherein the patients are ≥12 to <18 years old.

24. The method of claim 1, wherein the patients are any one of GINA step 1-5 patients.

25. The method of claim 1, wherein the wherein the fluticasone propionate is administered at a delivered dose of 22-59 mcg per inhalation and the albuterol sulfate is administered at a delivered dose of 92-124 mcg per inhalation.

26. The method of claim 25, wherein the fluticasone propionate is administered at a delivered dose of 23-56 mcg per inhalation and the albuterol sulfate is administered at a delivered dose of 97-119 mcg per inhalation.

27. The method of claim 26, wherein the fluticasone propionate is administered at a delivered dose of 25-54 mcg per inhalation and the albuterol sulfate is administered at a delivered dose of 103-113 mcg per inhalation.

28. The method of claim 27, wherein the fluticasone propionate is administered at a delivered dose of 26 or 51 mcg per inhalation and the albuterol sulfate is administered at a delivered dose of 108 mcg per inhalation.

29. The method of claim 1, wherein the fluticasone propionate is administered at a metered dose of 26-63 mcg per inhalation and the albuterol sulfate is administered at a metered dose of 99-135 mcg per inhalation.

30. The method of claim 29, wherein the fluticasone propionate is administered at a metered dose of 27-61 mcg per inhalation and the albuterol sulfate is administered at a metered dose of 105-129 mcg per inhalation.

31. The method of claim 30, wherein the fluticasone propionate is administered at a metered dose of 29-58 mcg per inhalation and the albuterol sulfate is administered at a metered dose of 111-123 mcg per inhalation.

32. The method of claim 31, wherein the fluticasone propionate is administered at a metered dose of 30 or 55 mcg per inhalation and the albuterol sulfate is administered at a metered dose of 117 mcg per inhalation.

33. The method of claim 1, wherein the fixed dose combination of fluticasone propionate and albuterol sulfate is administered at a maximum daily dose that does not exceed 12 inhalations.

34. The method of claim 1, wherein fixed-dose dry powder inhalation composition consists of fluticasone propionate, albuterol sulfate and alpha lactose monohydrate.
